# EUROPEAN PATENT SPECIFICATION

(11) **EP 3 438 645 B1**
(45) Date of publication and mention of the grant of the patent: **21.12.2022**
(21) Application number: 16897214.9
(22) Date of filing: 29.11.2016
(51) Int. Cl.: G01N 21/03, G01N 21/17, G01N 33/66, G01N 33/72, B01L 3/00, G01N 21/75

(54) **CHECK CASSETTE, MEASUREMENT DEVICE, SYSTEM FOR COMPENSATING FOR LIGHT INTENSITY OF LIGHT SOURCE FOR MEASUREMENT DEVICE, METHOD FOR COMPENSATING FOR LIGHT INTENSITY OF LIGHT SOURCE FOR MEASUREMENT DEVICE, AND RECORDING MEDIUM**
PRÜFUNGSKASSETTE, MESSVORRICHTUNG, SYSTEM ZUR KOMPENSATION DER LICHTINTENSITÄT EINER LICHTQUELLE FÜR DIE MESSVORRICHTUNG, VERFAHREN ZUR KOMPENSATION DER LICHTINTENSITÄT EINER LICHTQUELLE FÜR DIE MESSVORRICHTUNG UND AUFZEICHNUNGSMEDIUM
CASSETTE DE VÉRIFICATION, DISPOSITIF DE MESURE, SYSTÈME DE COMPENSATION D'INTENSITÉ LUMINEUSE DE SOURCE DE LUMIÈRE POUR DISPOSITIF DE MESURE, PROCÉDÉ DE COMPENSATION D'INTENSITÉ LUMINEUSE DE SOURCE DE LUMIÈRE POUR DISPOSITIF DE MESURE, ET SUPPORT D'ENREGISTREMENT

(30) Priority: 30.03.2016 KR 20160038154; 30.03.2016 KR 20160038155; 30.03.2016 KR 20160038157
(43) Date of publication of application: 06.02.2019
(73) Proprietor: OSANG HEALTHCARE CO., LTD., Anyang-si, Gyeonggi-do 14040 (KR)
(72) Inventor: KIM, Won Dong, Anyang-si Gyeonggi-do 14071 (KR); LEE, Seok Ki, Anyang-si Gyeonggi-do 14052 (KR); LEE, Jong Jin, Seoul 07064 (KR); HONG, Joo Pyo, Seoul 02517 (KR)
(74) Representative: Taor, Simon Edward William
(86) International application number: PCT/KR2016/013834
(87) International publication number: WO 2017/171194

(56) References cited:
- WO-A1-2013/086230
- WO-A2-2004/079342
- JP-A- 2011 196 790
- KR-A-101990 017 028
- US-A1- 2009 219 537
- US-A1- 2014 362 381
- Easy A1C, Manual of HbA1c Measuring Device, 2 December 2013 (2013-12-02), Retrieved from the Internet: URL:http://www. infopia21.com/product/manual/[% EA %B5%AD%EB%82%B4]% EC %9D%B4% EC %A7% 80% EC %97%90% EC %9D%B4% EC %9B%90% EC %94%AS Ariylyzer_ %EB%A7%A4% EB%89%B4% EC %96%BC(Revo2) _ 201 31211 %20o.pdf [retrieved on 2017-03-10]

## Description

### [Technical Field]

The present disclosure relates to a check cassette, measurement device, system for compensating for light intensity of a light source for the measurement device, method for compensating for the light intensity of the light source for the measurement device, and recording medium, and more particularly, to a check cassette, measurement device, system for compensating for light intensity of a light source for the measurement device, method for compensating for the light intensity of the light source for the measurement device, and recording medium capable of compensating for a change in light intensity of the light source in accordance with use of the measurement device, which is configured to measure the amount of glycated hemoglobin, so that accurate measurement is realized.

### [Background Art]

In recent years, in a medical diagnosis and therapeutic area through drugs, concentration measurements of an analyte regarding anesthetics or harmful chemical substances have been usefully used.

Among the above, concentration measurement of a biopsy sample used in a medical diagnosis and therapeutic field has gained more interest continuously with an increase in human desire to be liberated from various diseases.

Particularly, since a glycated hemoglobin test which is capable of measuring blood glucose in relation to diabetes is able to find out an average blood glucose level during a relatively long period of time with one measurement, an interest on the glycated hemoglobin test has been increased.

Hemoglobin Alc (HbA1c) is also referred to as glycated hemoglobin, which is one type of hemoglobin present in red blood cells of humans. When a glucose level in blood increases, some of the glucose in the blood binds to hemoglobin.

Hemoglobin which is bound to glucose in this way is referred to as glycated hemoglobin. A blood glucose level can be measured through such a glycated hemoglobin test. The glycated hemoglobin test has an advantage of being able to be conducted by collecting blood regardless of mealtime.

A device capable of measuring the amount of glycated hemoglobin has been disclosed in Korean Patent Registration No. KR10-0798471. In the case of Korean Patent Registration No. KR10-0798471, a measurement cassette which holds blood is inserted into a measurement device so as to measure the amount of glycated hemoglobin in blood.

In the case of the measurement device according to Korean Patent Registration No. KR10-0798471, there is a problem in that accuracy cannot be guaranteed in a process of measuring the amount of glycated hemoglobin.

In other words, the measurement device measures the amount of glycated hemoglobin using a reflection property of light irradiated from a light source, but since an aspect that light intensity of the light source is changed, e.g., the light intensity changes with time or the light intensity increases due to introduction of disturbance light, is not taken into consideration while measuring the amount of glycated hemoglobin, a problem occurs in terms of accuracy of a measured result.

Consequently, in measuring the amount of glycated hemoglobin in blood, research that allows accuracy of a measurement to be guaranteed by compensating for a change in light intensity of a light source in accordance with use of a measurement device is in urgent need.

WO 2004/079342 A2 relates to a method for normalizing for variations in signal intensity observed in a biomolecular binding assay carried out in a flow cell cartridge.

US 2009/219537 A1 relates to a method and apparatus for measuring properties of an unknown sample.

According to an aspect of the present invention, there is provided a system according to claim 1. According to another aspect of the present invention, there is provided a method according to claim 8.

It is an object of the present disclosure to provide a check cassette, measurement device, system for compensating for light intensity of a light source for the measurement device, method for compensating for the light intensity of the light source for the measurement device, and recording medium capable of compensating for a change in light intensity of the light source in accordance with use of the measurement device, which is configured to measure the amount of glycated hemoglobin, so that accurate measurement is realized and capable of simply realizing processes of inserting and separating a measurement cassette into and from the measurement device so that user convenience is maximized.

### [Technical Solution]

A check cassette according to an embodiment of the present disclosure is a cassette which is used in a measurement device which allows an analyte of a biopsy sample to be measured on the basis of reaction properties of light irradiated from a light source including at least one of reflection, absorption, and transmission of the light (the measurement device provides an inner space into which a measurement cassette, in which the biopsy sample and at least one reagent react with each other, is inserted, and after the measurement cassette is inserted into the inner space, the analyte of the biopsy sample is measured on the basis of pieces of reaction property information of the light irradiated from the light source regarding a substance that results from the reaction between the biopsy sample and the at least one reagent) so that a change in light intensity of the light source in accordance with use of the measurement device is compensated for, the check cassette including a first surface which has a first reaction surface having a first specific reaction property regarding specific light, and a second surface which is disposed opposite the first surface and has a second reaction surface having a second specific reaction property regarding the specific light, wherein, in a case in which the check cassette is inserted into the inner space in a first state, the first surface becomes a surface that is located closer to the light source than the second surface and allows first reaction property information of light irradiated from the light source due to the first reaction surface to be extracted by the measurement device, and in a case in which the measurement cassette is inserted into the inner space in a second state, which differs from the first state, the second surface becomes a surface that is located closer to the light source than the first surface and allows second reaction property information of light irradiated from the light source due to the second reaction surface to be extracted by the measurement device.

A position of the first reaction surface in the case in which the check cassette according to an embodiment of the present disclosure is inserted into the inner space in the first state may be the same as a position of the second reaction surface in the case in which the check cassette is inserted into the inner space in the second state.

A position of the first reaction surface with respect to the first surface of the check cassette according to an embodiment of the present disclosure may be the same as a position of the second reaction surface with respect to the second surface.

A position of the first reaction surface with respect to the first surface of the check cassette according to an embodiment of the present disclosure may be leaned toward one side with respect to a direction in which the check cassette is inserted into the inner space in the first state.

A position of the second reaction surface with respect to the second surface of the check cassette according to an embodiment of the present disclosure may be leaned toward one side with respect to a direction in which the check cassette is inserted into the inner space in the second state.

The second state of the check cassette according to an embodiment of the present disclosure may be a state in which the check cassette is rotated 180° from the first state with respect to a direction in which the check cassette is inserted into the inner space in the first state.

In a case in which the check cassette is inserted into the inner space in the first state, the second reaction surface of the check cassette according to an embodiment of the present disclosure does not exhibit a reaction property regarding light irradiated from the light source, and in a case in which the check cassette is inserted into the inner space in the second state, which differs from the first state, the first reaction surface does not exhibit a reaction property regarding light irradiated from the light source.

A measurement device according to another embodiment of the present disclosure is a measurement device which provides an inner space into which a measurement cassette, in which a biopsy sample and at least one reagent react with each other, is inserted only in a specific state, and after the measurement cassette is inserted into the inner space, allows an analyte of the biopsy sample to be measured on the basis of pieces of reaction property information of light irradiated from a light source including at least one of reflection, absorption, and transmission of the light regarding a substance that results from the reaction between the biopsy sample and the at least one reagent, wherein, in a case in which a check cassette is inserted into the inner space in a first state, the measurement device extracts first reaction property information of light irradiated from the light source due to a first reaction surface of a first surface which is located closer to the light source than a second surface, in a case in which the check cassette is inserted into the inner space in a second state, which differs from the first state, the measurement device extracts second reaction property information of light irradiated from the light source due to a second reaction surface of a second surface which is located closer to the light source than the first surface, and a change in light intensity of the light source in accordance with use of the measurement device is compensated for on the basis of the extracted first reaction property information and second reaction property information.

The measurement device according to another embodiment of the present disclosure may further include a measurement cassette guide portion configured to allow the measurement cassette to be inserted into the inner space only in a specific state, and in the cases in which the check cassette is inserted into the inner space in the first state and the second state, the measurement cassette guide portion may not interfere with the check cassette.

A system for compensating for light intensity of a light source for a measurement device according to still another embodiment of the present disclosure is a system which includes a measurement device which allows an analyte of a biopsy sample to be measured on the basis of reaction properties of light irradiated from the light source (the measurement device provides an inner space into which a measurement cassette, in which the biopsy sample and at least one reagent react with each other, is inserted, and after the measurement cassette is inserted into the inner space, the analyte of the biopsy sample is measured on the basis of pieces of reaction property information of light irradiated from the light source including at least one of reflection, absorption, and transmission of the light regarding a substance that results from the reaction between the biopsy sample and the at least one reagent) and a check cassette which is used in the measurement device so as to compensate for a change in light intensity of the light source in accordance with use of the measurement device, wherein the measurement device has the light source disposed at a first position in the inner space, the check cassette has a first surface which has a first reaction surface having a first specific reaction property regarding specific light and a second surface which is disposed opposite the first surface and has a second reaction surface having a second specific reaction property regarding the specific light, in a case in which the check cassette is inserted into the inner space in a first state, the measurement device extracts first reaction property information of light irradiated from the light source due to the first reaction surface of the first surface which is located closer to the light source than the second surface, in a case in which the check cassette is inserted into the inner space in a second state, which differs from the first state, the measurement device extracts second reaction property information of light irradiated from the light source due to the second reaction surface of the second surface which is located closer to the light source than the first surface, and a change in light intensity of the light source in accordance with use of the measurement device is compensated for on the basis of the extracted first reaction property information and second reaction property information.

The second state of the system for compensating for light intensity of a light source for a measurement device according to yet another embodiment of the present disclosure may be a state in which the check cassette is rotated 180° from the first state with respect to a direction in which the check cassette is inserted into the inner space in the first state.

The measurement device of the system for compensating for light intensity of a light source for a measurement device according to yet another embodiment of the present disclosure may have a measurement cassette guide portion configured to allow the measurement cassette to be inserted into the inner space only in a specific state, and in the cases in which the check cassette is inserted into the inner space in the first state and the second state, the measurement cassette guide portion may not interfere with the check cassette.

A method for compensating for light intensity of a light source for a measurement device according to yet another embodiment of the present disclosure is a method for compensating for a change in light intensity of the light source in accordance with use of the measurement device which allows an analyte of a biopsy sample to be measured on the basis of reaction properties of light irradiated from the light source including at least one of reflection, absorption, and transmission of the light (the measurement device provides an inner space into which a measurement cassette, in which the biopsy sample and at least one reagent react with each other, is inserted, and after the measurement cassette is inserted into the inner space, the analyte of the biopsy sample is measured on the basis of pieces of reaction property information of the light irradiated from the light source regarding a substance that results from the reaction between the biopsy sample and the at least one reagent), the method including a first step in which a check cassette, which includes a first surface which has a first reaction surface having a first specific reaction property regarding specific light and a second surface which is disposed opposite the first surface and has a second reaction surface having a second specific reaction property regarding the specific light, is inserted into the inner space in a first state, a second step in which, when the check cassette is inserted into the inner space in the first state, first reaction property information of light irradiated from the light source due to the first reaction surface is extracted, a third step in which the check cassette is separated from the inner space, a fourth step in which the check cassette is inserted into the inner space in a second state, which differs from the first state, a fifth state in which, when the check cassette is inserted into the inner space in the second state, second reaction property information of light irradiated from the light source due to the second reaction surface is extracted, and a sixth step in which a change in light intensity of the light source in accordance with use of the measurement device is compensated for on the basis of the extracted first reaction property information and second reaction property information.

The second state of the method for compensating for light intensity of a light source for a measurement device according to yet another embodiment of the present disclosure may be a state in which the check cassette is rotated 180° from the first state with respect to a direction in which the check cassette is inserted into the inner space in the first state.

The first step of the method for compensating for light intensity of a light source for a measurement device according to yet another embodiment of the present disclosure may include, when the check cassette does not satisfy a predetermined first condition (at least one of a condition related to whether the check cassette is completely inserted into the inner space in the first state, a condition related to time taken for the check cassette to be completely inserted into the inner space, and a condition related to time during which the check cassette remains completely inserted into the inner space), outputting a signal that allows the non-satisfaction state to be identified.

The third step of the method for compensating for light intensity of a light source for a measurement device according to yet another embodiment of the present disclosure may include, when the check cassette does not satisfy a predetermined second condition (at least one of a condition related to whether the check cassette is completely separated from the inner space and a condition related to time taken for the check cassette to be completely separated), outputting a signal that allows the non-satisfaction state to be identified.

The fourth step of the method for compensating for light intensity of a light source for a measurement device according to yet another embodiment of the present disclosure may include, when the check cassette does not satisfy a predetermined third condition (at least one of a condition related to whether the check cassette is completely inserted into the inner space in the second state, a condition related to time taken for the check cassette to be completely inserted into the inner space, and a condition related to time during which the check cassette remains completely inserted into the inner space), outputting a signal that allows the non-satisfaction state to be identified.

The first step of the method for compensating for light intensity of a light source for a measurement device according to yet another embodiment of the present disclosure may include selecting a mode of the measurement device in order to insert the check cassette into the inner space in the first state.

A recording medium according to yet another embodiment of the present disclosure records a program for performing a method for compensating for a light source for a measurement device.

### [Advantageous Effects]

A check cassette, measurement device, system for compensating for light intensity of a light source for the measurement device, method for compensating for the light intensity of the light source for the measurement device, and recording medium according to the present disclosure can compensate for a change in light intensity of the light source in accordance with use of the measurement device configured to measure the amount of glycated hemoglobin so that accurate measurement is realized.

The present disclosure can simply realize processes of inserting and separating a measurement cassette into and from the measurement device so that user convenience is maximized.

### [Description of Drawings]

FIG. 1 is a schematic perspective view illustrating a measurement device according to an embodiment of the present disclosure.
FIG. 2 is a schematic perspective view illustrating a measurement cassette for inserting a biopsy sample to be measured by the measurement device according to FIG. 1.
FIGS. 3 to 8 are views for describing a process in which the measurement cassette is inserted into the measurement device according to FIG. 1.
FIG. 9 is a view for describing a process in which the measurement cassette is separated from the measurement device according to FIG. 1.
FIG. 10 is a schematic perspective view illustrating a system for compensating for light intensity of a light source for a measurement device according to another embodiment of the present disclosure.
FIG. 11 is a schematic perspective view illustrating a check cassette provided in the system for compensating for light intensity of a light source for a measurement device according to FIG. 10.
FIG. 12 is a flowchart for describing a method for compensating for light intensity of a light source by using the system for compensating for light intensity of a light source for a measurement device according to FIG. 10.
FIGS. 13 to 18 are views for describing each step according to FIG. 12 in detail.
FIG. 19 is a schematic perspective view illustrating the system for compensating for light intensity of a light source for a measurement device according to the present disclosure.
FIG. 20 is a flowchart for describing a method for compensating for light intensity of a light source for a measurement device by using a first check cassette and a second check cassette according to FIG. 19.
FIGS. 21 to 26 are views for describing each step according to FIG. 20 in detail.

### [Best Mode of the Invention]

A check cassette according to an embodiment of the present disclosure is a cassette which is used in a measurement device which allows an analyte of a biopsy sample to be measured on the basis of reaction properties of light irradiated from a light source including at least one of reflection, absorption, and transmission of the light (the measurement device provides an inner space into which a measurement cassette, in which the biopsy sample and at least one reagent react with each other, is inserted, and after the measurement cassette is inserted into the inner space, the analyte of the biopsy sample is measured on the basis of pieces of reaction property information of the light irradiated from the light source regarding a substance that results from the reaction between the biopsy sample and the at least one reagent) so that a change in light intensity of the light source in accordance with use of the measurement device is compensated for, the check cassette including a first surface which has a first reaction surface having a first specific reaction property regarding specific light, and a second surface which is disposed opposite the first surface and has a second reaction surface having a second specific reaction property regarding the specific light, wherein, in a case in which the check cassette is inserted into the inner space in a first state, the first surface becomes a surface that is located closer to the light source than the second surface and allows first reaction property information of light irradiated from the light source due to the first reaction surface to be extracted by the measurement device, and in a case in which the measurement cassette is inserted into the inner space in a second state, which differs from the first state, the second surface becomes a surface that is located closer to the light source than the first surface and allows second reaction property information of light irradiated from the light source due to the second reaction surface to be extracted by the measurement device.

### [Modes of the Invention]

Hereinafter, specific embodiments of the present disclosure will be described in detail with reference to the accompanying drawings. The scope of the invention is only limited by the appended claims.

### 1. Measurement cassette and measurement device

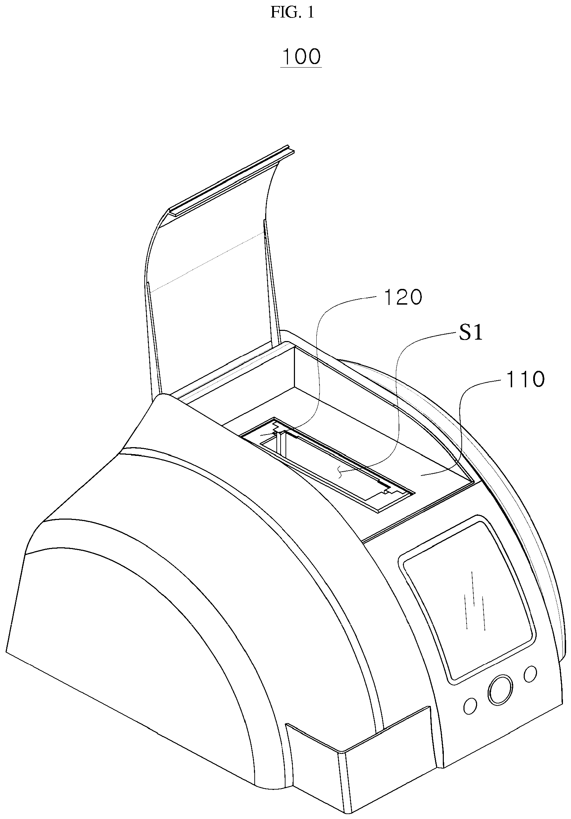

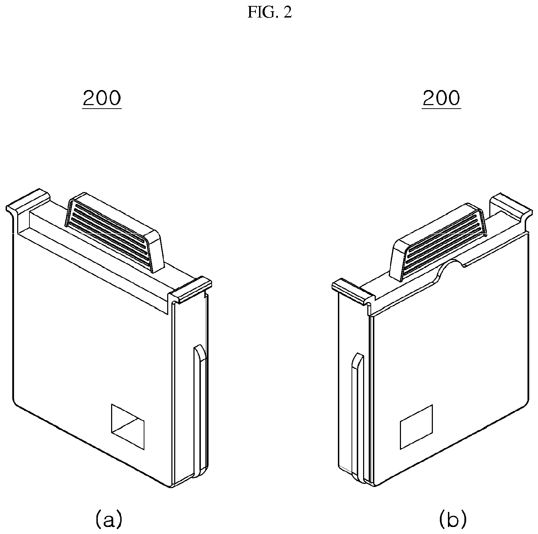

FIG. 1 is a schematic perspective view illustrating a measurement device according to an embodiment of the present disclosure, and FIG. 2 is a schematic perspective view illustrating a measurement cassette for inserting a biopsy sample to be measured by the measurement device according to FIG. 1.

Referring to FIGS. 1 and 2, a measurement device 100 according to an embodiment of the present disclosure is a device that allows an analyte of a biopsy sample to be measured on the basis of reaction properties of light irradiated from a light source including at least one of reflection, absorption, and transmission of the light. For example, the biopsy sample may be blood, and the analyte may be glycated hemoglobin.

When attempting to measure the amount of glycated hemoglobin, which is the analyte, in the blood, which is the biopsy sample, the measurement device 100 may use a property of hemoglobin specifically absorbing an optical signal having a specific frequency.

Specifically, the measurement device 100 may provide an inner space S1 into which a measurement cassette 200, in which the biopsy sample and at least one reagent react with each other, is inserted, and after the measurement cassette 200 is inserted into the inner space S1, the measurement device 100 may allow the analyte of the biopsy sample to be measured on the basis of pieces of reaction property information of light irradiated from the light source regarding a substance that results from the reaction between the biopsy sample and the at least one reagent.

When the measurement cassette 200, in which the biopsy sample and the at least one reagent are located, is inserted into the inner space S1, the measurement cassette 200 may be rotated clockwise or counterclockwise according to a predetermined pattern, and due to the rotation, the biopsy sample and the reagent may be stirred or moved together, and the amount of glycated hemoglobin in the biopsy sample may be measured.

Since a method of measuring glycated hemoglobin by the measurement device 100, a configuration of the measurement cassette 200, and the like have been already disclosed in Korean Patent Publication No. KR2010-0136744, detailed description thereof will be omitted.

In a case in which the measurement cassette 200 is inserted into the inner space S1 of the measurement device 100 so as to measure the analyte in the biopsy sample, that is, the amount of glycated hemoglobin in blood, the measurement cassette 200 has to be stably located at an optimal position regarding the relationship with the light source of the measurement device 100. The measurement device 100 according to the present disclosure includes a configuration for this, which will be described in detail below.

The measurement device 100 according to the present disclosure includes a configuration for preventing the measurement cassette 200 from being separated from the inner space S1 while the analyte in the biopsy sample is being measured using the measurement cassette 200 inserted into the inner space S1. The configuration will be described in detail below.

When the measurement of the analyte in the biopsy sample is completed, the measurement device 100 according to the present disclosure has to be separated from the inner space S1 easily and conveniently. The measurement device 100 includes a configuration for this, which will be described in detail below.

### 2. Processes in which measurement cassette is inserted into and separated from measurement device

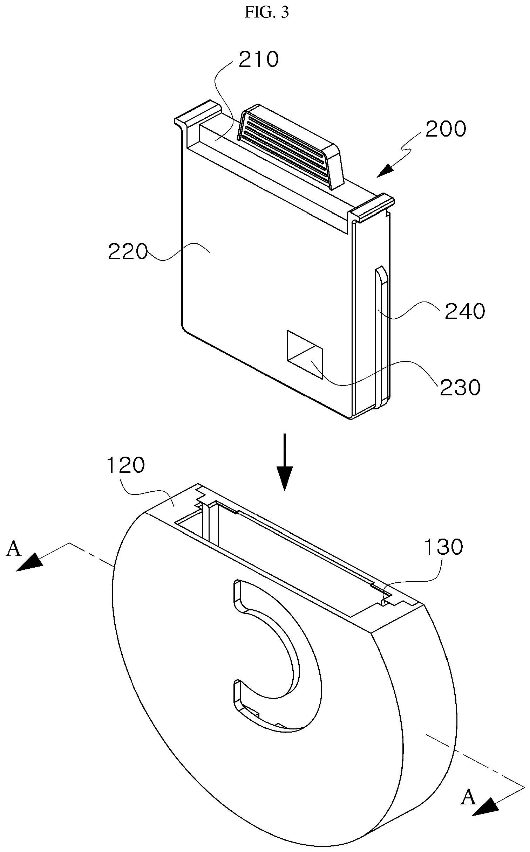

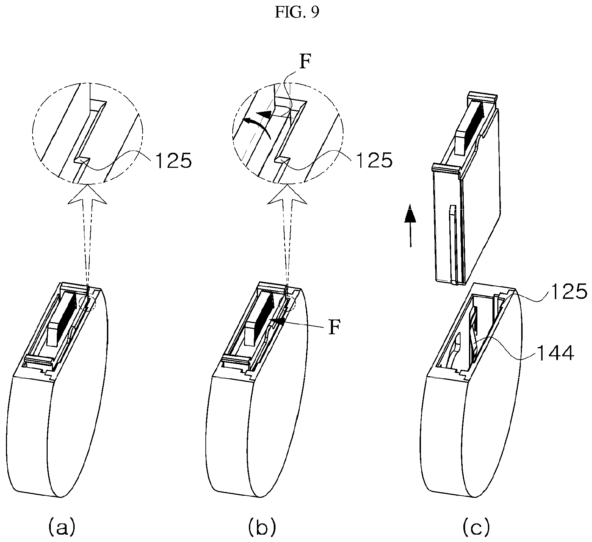

FIGS. 3 to 8 are views for describing a process in which the measurement cassette is inserted into the measurement device according to FIG. 1, and FIG. 9 is a view for describing a process in which the measurement cassette is separated from the measurement device according to FIG. 1.

First, referring to FIGS. 3 to 8, a process in which the measurement cassette 200 is inserted into the measurement device 100 (see FIG. 1) will be described.

Referring to FIG. 3, to measure the analyte of the biopsy sample, the measurement device 100 and the measurement cassette 200, which contains the biopsy sample and at least one reagent, are prepared.

The measurement cassette 200 may include a reagent container 210 in which the biopsy sample and the at least one reagent are stored and a cassette 220 configured to provide a space in which the biopsy sample and the at least one reagent react with each other after the reagent container 210 is inserted therein.

Hereinafter, the process in which the measurement cassette 200 is inserted into the measurement device 100 will be described using a case in which the measurement cassette 200 is inserted into the inner space S1 of the measurement device 100 while the reagent container 210 is inserted into the cassette 220 as an example. However, the present disclosure is not necessarily limited thereto, and may also include a case in which the reagent container 210 is inserted into the cassette 220 after the cassette 220 is completely inserted into the inner space S1 of the measurement device 100.

That is, the measurement cassette 200 described with reference to FIGS. 3 to 8 is not limited to the concept including the reagent container 210 and the cassette 220, and may be understood as referring to the cassette 220.

The measurement device 100 may include a main body portion 110 (see FIG. 1) which provides an exterior and has elements such as a light source, which allows an analyte of a biopsy sample to be measured, mounted thereon, and a defining portion 120 mounted on the main body portion 110 so as to define the inner space S1 into which the measurement cassette 200 is inserted.

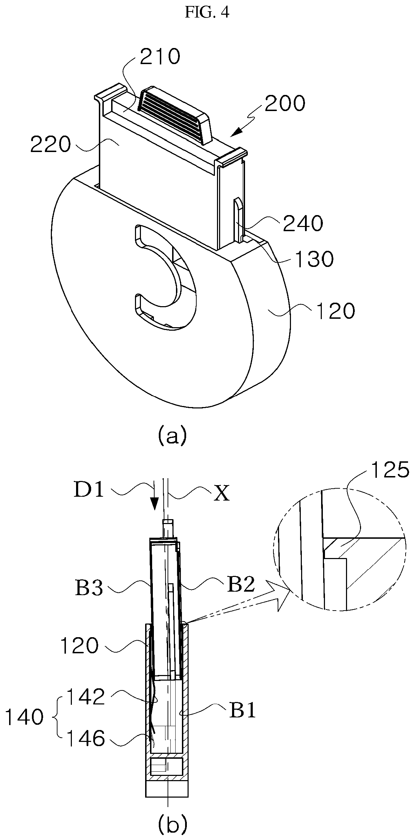

To measure the analyte in the biopsy sample, that is, the amount of glycated hemoglobin in the blood, as illustrated in FIG. 4, the measurement cassette 200, which is the cassette 220 into which the reagent container 210 is inserted, is inserted into the inner space S1.

In this case, the measurement cassette 200 has to be inserted into the inner space S1 only in a specific state. This is because a position of a measurement area 230 of the measurement cassette 200 inserted into the inner space S1 has to correspond to a position of the light source.

The measurement device 100 may have a measurement cassette guide portion 130 so as to allow the measurement cassette 200 to be inserted into the inner space S1 only in a specific state.

The measurement cassette guide portion 130 is an element of the defining portion 120 and may be formed to be depressed from an inner surface and leaned toward one direction.

The measurement cassette 200 may have a corresponding portion 240 which is formed to protrude to correspond to the measurement cassette guide portion 130.

As a result, in a case in which the measurement cassette 200 is inserted into the inner space S1, the measurement cassette 200 may be inserted into the inner space S1 only in a case in which the corresponding portion 240 is inserted into the measurement cassette guide portion 130, and insertion of the measurement cassette 200 into the inner space S1 becomes impossible in the opposite case.

However, the measurement cassette guide portion 130 does not have to be necessarily formed to be depressed and may also be formed to protrude. When the measurement cassette guide portion 130 is formed to protrude, the corresponding portion 240 may be formed to be depressed.

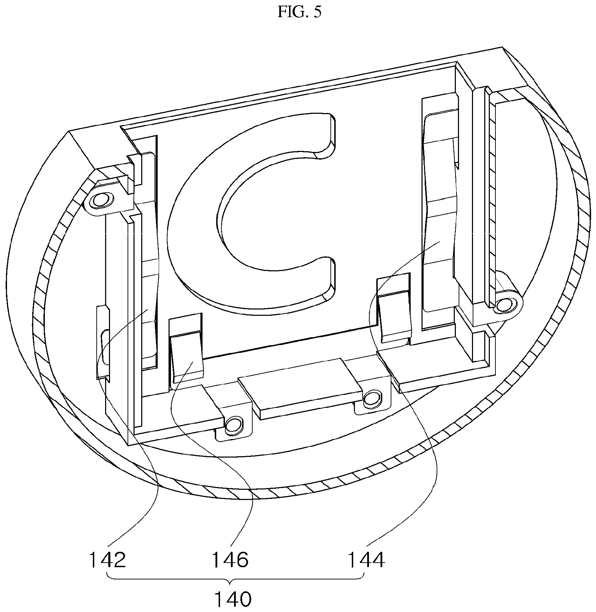

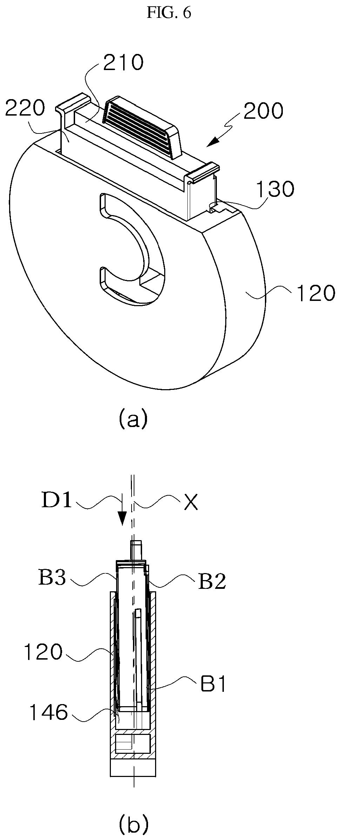

Referring to FIGS. 5 (cross-sectional view taken along line A-A of FIG. 3) and FIG. 6, in the process in which the measurement cassette 200 is inserted into the inner space S1, the measurement cassette 200 receives a pressing force due to a pressing portion 140.

The pressing portion 140 may allow the measurement cassette 200, which is mounted on the defining portion 120 and inserted into the inner space S1, to be stably located in the inner space S1. For this, the pressing portion 140 may provide a pressing force that allows the measurement cassette 200 inserted into the inner space S1 to be pressed toward one surface B1 of an inner surface of the defining portion 120.

Specifically, the pressing portion 140 may include a first pressing portion 142 and a second pressing portion 144 which are elastically deformed due to coming into contact with the other surface B3, which is a surface opposite one surface B2 of the measurement cassette 200 facing the one surface B1 of the inner surface of the defining portion 120, in a case in which the measurement cassette 200 is inserted into the inner space S1, and may further include a third pressing portion 136 configured to provide an additional pressing force to the other surface B3 of the measurement cassette 200 after the pressing force is provided to the other surface B3 of the measurement cassette 200 by the first pressing portion 142 and the second pressing portion 144.

The first pressing portion 142 and the second pressing portion 144 may be located at positions respectively corresponding to both ends of the measurement cassette 200. As illustrated in FIGS. 5 and 6, the first pressing portion 142 and the second pressing portion 144 are elastically deformed due to insertion of the measurement cassette 200 into the inner space S1 and apply a restoration force due to the elastic deformation to the measurement cassette 200.

When a pressing force having a predetermined magnitude is provided to the other surface B3 of the measurement cassette 200 by the first pressing portion 142 and the second pressing portion 144, a direction in which the measurement cassette 200 is inserted may be a direction D1 inclined in the inner space S1, and in this case, the one surface B2 of the measurement cassette 200 comes into contact with a contact portion 125 of the defining portion 120.

The contact portion 125 may be a type of locking step that protrudes from an upper end of the one surface B1 of the inner surface of the defining portion 120 toward the other surface of the inner surface.

In a case in which the measurement cassette 200 is inserted into the inner space S1 as the pressing force due to the first pressing portion 142 and the second pressing portion 144 is provided to the measurement cassette 200, the contact portion 125 may come into contact with the one surface B2 of the measurement cassette 200 facing the one surface B1 of the inner surface of the defining portion 120, and because of this, the direction in which the measurement cassette 200 is inserted may be the direction D1 inclined in the inner space S1.

In a case in which the measurement cassette 200 is inserted into the inner space S1, the first pressing portion 142 and the second pressing portion 144 may press the other surface B3 of the measurement cassette 200 so that the contact portion 125 and the one surface B2 of the measurement cassette 200 remain in contact with each other.

As a result, the contact portion 125 comes into contact with one surface of the measurement cassette 200 during the insertion of the measurement cassette 200 so that the measurement cassette 200 is inserted into the inner space S1 in the direction D1 inclined with respect to a vertical axis X, and to maintain such a state, the pressing portion 140 presses the other surface of the measurement cassette 200 due to the insertion of the measurement cassette 200.

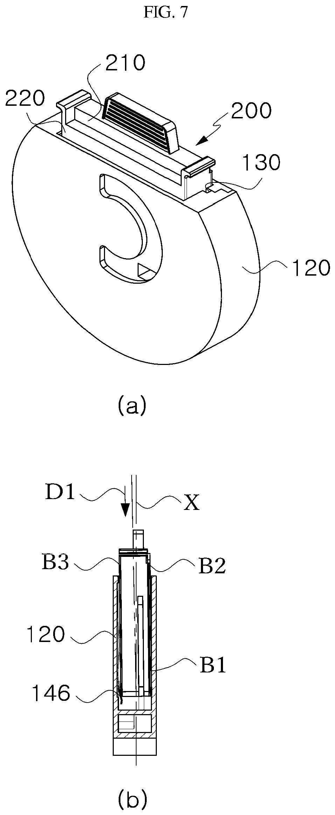

When the measurement cassette 200 is inserted into the inner space S1 so as to be tilted by the first pressing portion 142, the second pressing portion 144, and the contact portion 125, as illustrated in FIG. 7, the other surface of the measurement cassette 200 may receive an additional pressing force due to the third pressing portion 146.

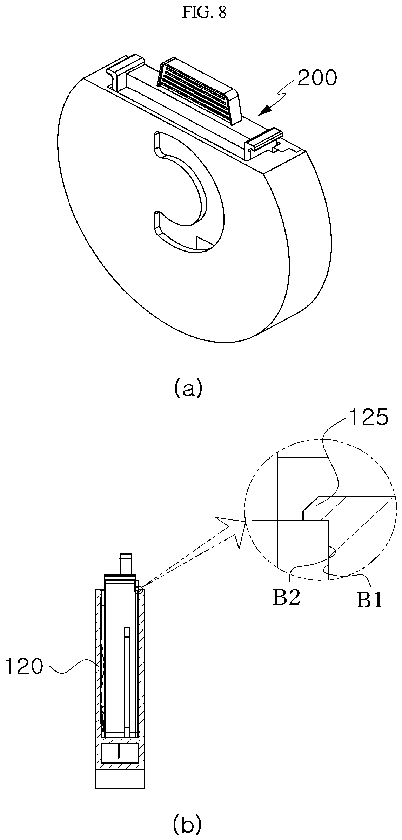

By the third pressing portion 146, the measurement cassette 200 may be guided to be stably located at an optimal position regarding the relationship with the light source of the measurement device 100. When insertion of the measurement cassette 200 into the inner space S1 is completed, as illustrated in FIG. 8, the measurement cassette 200 may be prevented from being deviated from the inner space S1.

In other words, when insertion of the measurement cassette 200 into the inner space S1 is completed, deviation of the measurement cassette 200 from the inner space S1 may be prevented by the contact portion 125. This is because an upper end of the one surface B2 of the measurement cassette 200 is locked to the contact portion 125.

When insertion of the measurement cassette 200 into the inner space S1 is completed, the pressing portion 140 may provide the pressing force due to the restoration force due to the elastic deformation to the other surface B3 of the measurement cassette 200 and allow the upper end of the one surface B2 of the measurement cassette 200 to remain locked to the contact portion 125. Because of this, separation of the measurement cassette 200 from the inner space S1 may be prevented while the analyte in the biopsy sample is being measured using the measurement cassette 200 inserted into the inner space S1.

When the measurement cassette 200, in which the biopsy sample and the at least one reagent are located, is completely inserted into the inner space S1 of the measurement device 100 as illustrated in FIG. 8, the measurement cassette 200 may be rotated clockwise or counterclockwise according to a predetermined pattern. Because of this, the biopsy sample and the reagent may be stirred or moved together, and the amount of glycated hemoglobin in the biopsy sample may be measured.

Due to the measurement of the amount of glycated hemoglobin, the measurement device 100 may also extract a proportion of glycated hemoglobin with respect to the total hemoglobin.

After the amount of glycated hemoglobin in the biopsy sample is measured by the measurement device 100, the measurement cassette 200 has to be separated from the inner space S1. The separation may be realized by an external force of a user applied to the one surface B2 of the measurement cassette 200 as illustrated in FIG. 9.

When an external force F of the user is applied to the one surface B2 of the measurement cassette 200, the upper end of the one surface B2 of the measurement cassette 200 may be spaced apart from the contact portion 125, and the measurement cassette 200 is naturally separated from the inner space S1 when the user pulls the measurement cassette 200 upward in the above state.

In this case, note that, of course, the external force F of the user has to be greater than the pressing force provided to the other surface B3 of the measurement cassette 200 by the pressing portion 140.

As described above, when measurement of the analyte in the biopsy sample is completed by the measurement device 100 according to the present disclosure, the measurement cassette 200 may be easily and conveniently separated from the inner space S1 by the external force F of the user.

3. Check cassette for compensating for change in light intensity of light source of measurement device, measurement device, system for compensating for light intensity of light source for measurement device, method for compensating for light intensity of light source for measurement device, and recording medium in which program for performing this method is recorded

### (1) First embodiment

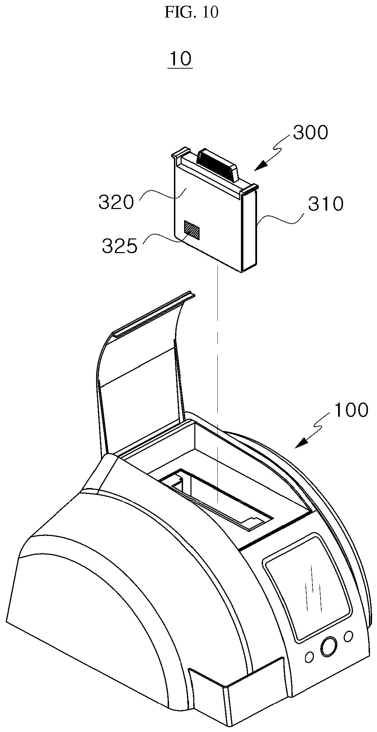

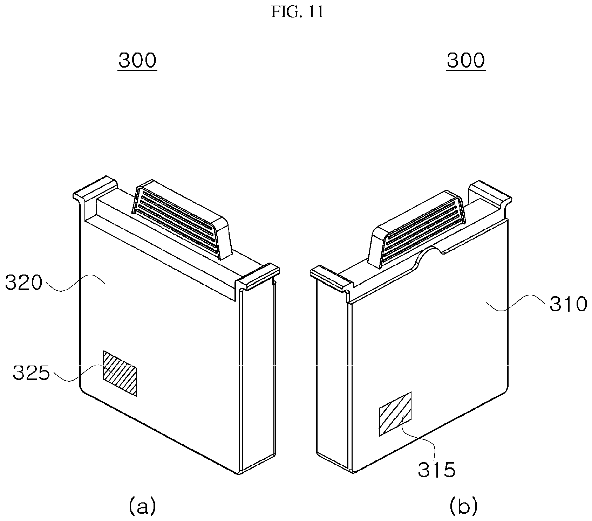

FIG. 10 is a schematic perspective view illustrating a system for compensating for light intensity of a light source for a measurement device according to another embodiment of the present disclosure, and FIG. 11 is a schematic perspective view illustrating a check cassette provided in the system for compensating for light intensity of a light source for a measurement device according to FIG. 10.

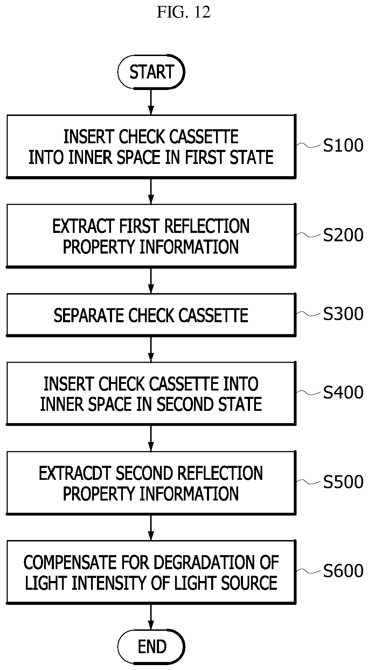

FIG. 12 is a flowchart for describing a method for compensating for light intensity of a light source by using the system for compensating for light intensity of a light source for a measurement device according to FIG. 10, and FIGS. 13 to 18 are views for describing each step according to FIG. 12 in detail.

A measurement device 100 according to the present disclosure is a device which allows an analyte of a biopsy sample to be measured on the basis of a reaction property of light irradiated from a light source. The measurement device 100 essentially includes a light source. For example, the measurement device 100 may include a light-emitting element and a light-receiving element such as a photo diode.

In other words, the measurement device 100 measures the amount of glycated hemoglobin in the blood from light intensity of light that reaches the light-receiving element without being absorbed specifically by glycated hemoglobin in the blood from among light intensities of light having a specific frequency that is irradiated from the light-emitting element.

In this case, to measure the amount of glycated hemoglobin from light intensity of light irradiated by the light-receiving element and light intensity of light that reaches the light-receiving element, the relationship between the light intensity of light irradiated from the light-emitting element and the light intensity of light that reaches the light-receiving element has to be predefined in accordance with the amount of glycated hemoglobin.

However, due to a property of a light source, that is, a property of the light-emitting element, the light intensity of irradiated light is changed inevitably, i.e. degraded or increased due to introduction of disturbance light, in accordance with use of the measurement device 100. Because of this, the relationship between the light intensity of light irradiated from the light-emitting element and the light intensity of light that reaches the light-receiving element in accordance with the amount of glycated hemoglobin has to be compensated. An accurate measurement may become possible despite repeated uses of the measurement device 100 only when the compensation is performed.

Due to the above reason, the present disclosure provides a system and method for compensating for a change in light intensity of a light source in accordance with use of the measurement device 100, and a check cassette, a measurement device, and the like provided in the system.

First, referring to FIGS. 10 and 11, a system 10 for compensating for light intensity of a light source for a measurement device according to the present disclosure may include the measurement device 100, which has been described above with reference to FIGS. 1 to 9, and a check cassette 300.

The check cassette 300 is a cassette for compensating for a change in light intensity of a light source of the measurement device 100, unlike the measurement cassette 200 which has been described above with reference to FIGS. 1 to 9. The check cassette 300 includes a first surface 310 and a second surface 320 disposed opposite the first surface 310.

The first surface 310 includes a first reaction surface 315 having a first specific reaction property regarding specific light, and the second surface 320 includes a second reaction surface 325 having a second specific reaction property regarding the specific light.

In this case, the first specific reaction property may be a property that defines the relationship between light intensity of specific light irradiated by a specific light-emitting element and light intensity of light that reaches a light-receiving element after being reacted due to the first reaction surface 315. The second specific reaction property may be a property that defines the relationship between light intensity of specific light irradiated by the specific light-emitting element and light intensity of light that reaches the light-receiving element after being reacted due to the second reaction surface.

Hereinafter, a method for compensating for light intensity of a light source using the system 10 for compensating for light intensity of a light source for a measurement device will be described in detail.

Referring to FIG. 12, a method for compensating for light intensity of a light source using the system for compensating for light intensity of a light source for a measurement device according to the present disclosure includes a first step (S100) in which the check cassette 300 is inserted into the inner space S1 of the measurement device 100 in a first state, a second step (S200) in which first reaction property information of light irradiated from the light source due to the first reaction surface 310 is extracted, a third step (S300) in which the check cassette 300 is separated from the inner space S1, a fourth step (S400) in which the check cassette 300 is inserted into the inner space S1 in a second state, a fifth step (S500) in which second reaction property information of light irradiated from the light source due to the second reaction surface 320 is extracted, and a sixth step (S600) in which a change in light intensity of the light source is compensated for.

In this case, the first state may be a state in which the check cassette 300 is rotated 180° from the second state with respect to a direction in which the check cassette 300 is inserted into the inner space S1, and the second state may be a state in which the check cassette 300 is rotated 180° from the first state with respect to the direction in which the check cassette 300 is inserted into the inner space S1.

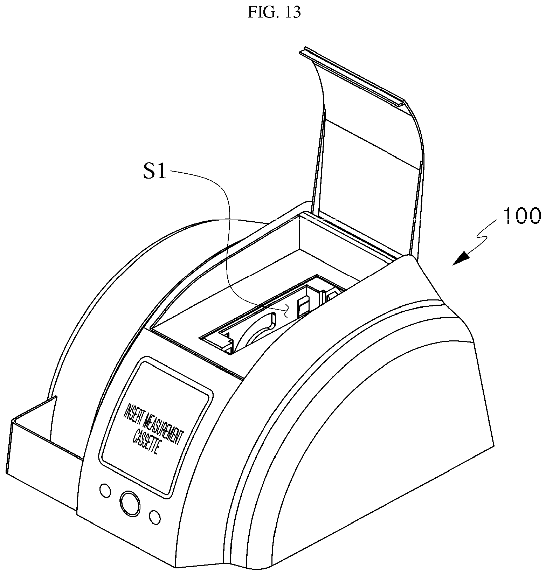

Hereinafter, each step will be described in more detail with reference to FIGS. 13 to 18.

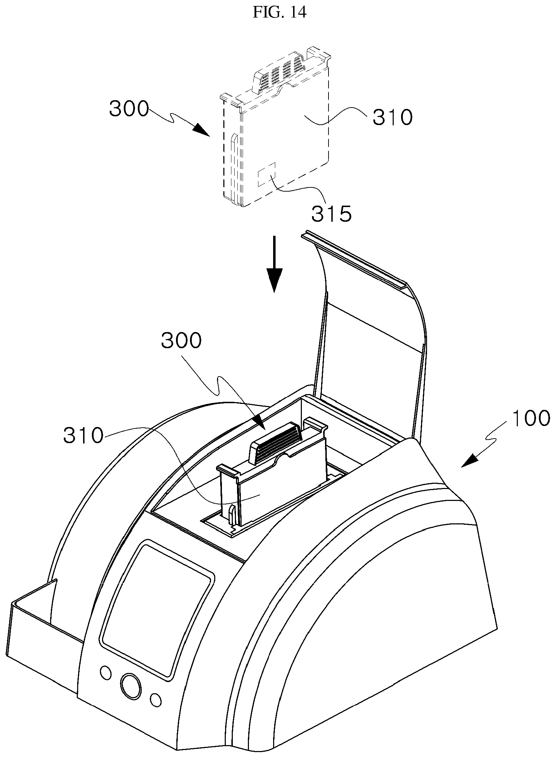

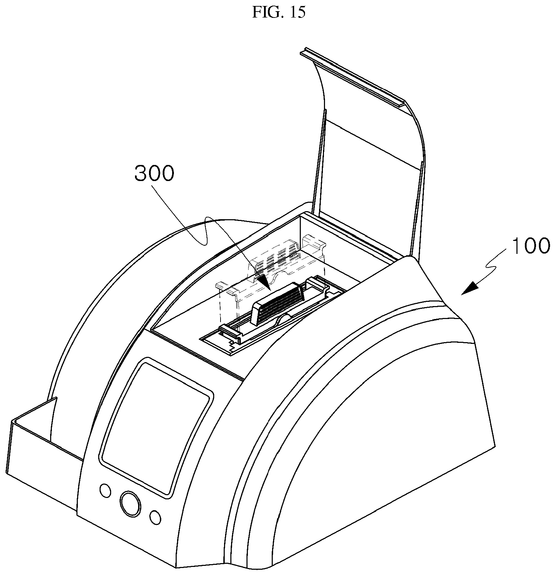

Referring to FIGS. 14 and 15, the first step (S100) in which the check cassette 300, which includes the first surface 310 which has the first reaction surface 315 having the first specific reaction property regarding specific light and the second surface 320 which is disposed opposite the first surface 310 and has the second reaction surface 325 having the second specific reaction property regarding the specific light, is inserted into the inner space S1 in the first state may be performed.

In this case, as illustrated in FIG. 13, to insert the check cassette 300 into the inner space S1 in the first state, selecting a mode of the measurement device 100 and displaying a point in time at which the check cassette 300 is inserted may be performed in the first step (S100), wherein the mode may include a measurement mode and a check mode.

The measurement mode may be a mode which allows an analyte in the biopsy sample to be measured using the measurement cassette (see FIGS. 1 to 9) in which the biopsy sample and the at least one reagent react with each other, and the check mode may be a mode for compensating for a change in light intensity of the light source of the measurement device 100 using the check cassette 300.

After the check mode is selected, the measurement device 100 may inform the user of the fact that a current point in time is a point in time at which the check cassette 300 has to be inserted into the inner space S1.

The first step (S100) has been described above as including the selecting of the mode and the displaying of the point in time at which the check cassette 300 is inserted, but such steps are not necessarily required.

In inserting the check cassette 300 into the inner space S1 in the first state, the second step (S200) may be performed only when the check cassette 300 satisfies a predetermined first condition. The predetermined first condition may include various conditions.

For example, the predetermined first condition may be at least one of a condition related to whether the check cassette 300 is completely inserted into the inner space S1 in the first state, a condition related to time taken for the check cassette 300 to be completely inserted into the inner space S1, and a condition related to time during which the check cassette 300 remains completely inserted into the inner space S1.

First, in a case in which the predetermined first condition is the condition related to whether the check cassette 300 is completely inserted into the inner space S1 in the first state, the check cassette 300 has to reach a state illustrated in FIG. 15 in order to satisfy the predetermined first condition.

Second, in a case in which the predetermined first condition is the condition related to time taken for the check cassette 300 to be completely inserted into the inner space S1, time taken for the check cassette 300 to reach the state illustrated in FIG. 15 has to be within a predetermined first time in order to satisfy the predetermined first condition.

For example, in a case in which the predetermined first time is five seconds, when the check cassette 300 is not completely inserted into the inner space S1 until five seconds passes from a point in time at which the check cassette 300 has to be inserted into the inner space S1 after the check mode is selected, the predetermined first condition is not satisfied.

In this case, the measurement device 100 may output a signal that allows the non-satisfaction state to be identified visually or aurally by a controller.

Third, in a case in which the predetermined first condition is the condition related to time during which the check cassette 300 remains completely inserted into the inner space S1, the check cassette 300 has to remain in the state illustrated in FIG. 15 during a predetermined second time in order to satisfy the predetermined first condition.

For example, in a case in which the predetermined second time is five seconds, when the check cassette 300 does not remain completely inserted into the inner space S1 for at least five seconds, the predetermined first condition is not satisfied.

In this case, the measurement device 100 may output a signal that allows the non-satisfaction state to be identified visually or aurally by the controller.

To determine the above-described predetermined first condition, the measurement device 100 according to the present disclosure may include a detection means such as a sensor capable of sensing whether the check cassette 300 has been completely inserted into the inner space S1, a timer, and the like.

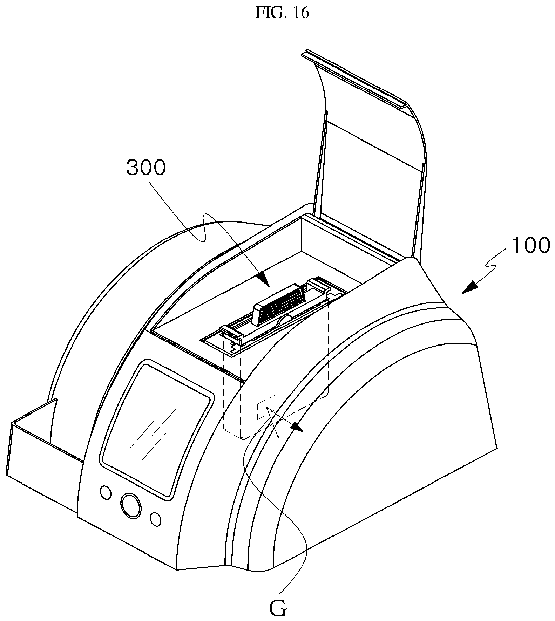

Referring to FIG. 16, after the check cassette 300 is inserted into the inner space S1 in the first state, the second step (S200) in which first reaction property information of light G irradiated from the light source due to the first reaction surface 315 is extracted may be performed.

The second step (S200) may be a step in which the first surface 310 becomes a surface located closer to the light source than the second surface such that the first reaction property information of light irradiated from the light source due to the first reaction surface 315 is extracted by the measurement device 100.

Specifically, when the check cassette 300 satisfies the predetermined first condition, the controller may allow light to be irradiated from the light-emitting element toward the first reaction surface 315, and extract the first reaction property information through light intensity of light that is reacted due to the first reaction surface 315 and then reaches the light-receiving element.

In this case, as described above, the first reaction surface 315 has a first specific reaction property regarding specific light. In a case in which light irradiated from the light-emitting element has the same frequency and light intensity as those of specific light, the first specific reaction property and the first reaction property have to be the same.

In other words, in a case in which the specific light is the light of the light source, when the first reaction property differs from the first specific reaction property, this signifies that light intensity of the light source has changed.

In the case in which the check cassette 300 is inserted into the inner space S1 in the first state, the second reaction surface 325 of the second surface 320 may not exhibit a reaction property regarding the light G irradiated from the light source.

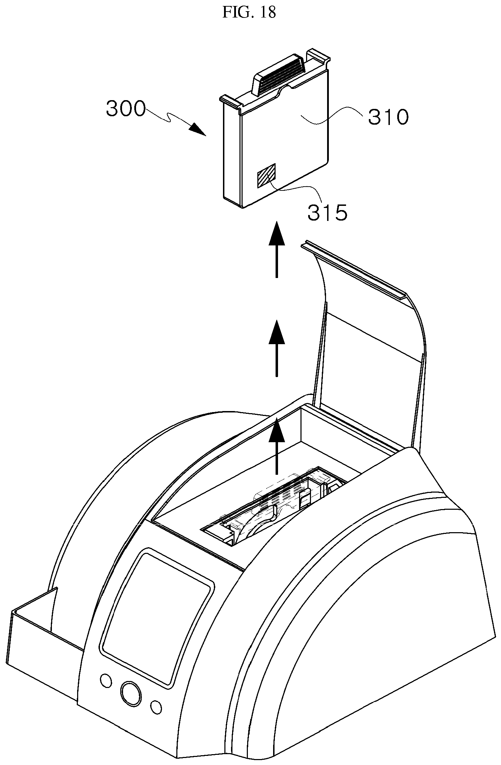

Referring to FIG. 18, after the first reaction property information of the light irradiated from the light source due to the first reaction surface 315 is extracted, the third step (S300) in which the check cassette 300 is separated from the inner space S1 may be performed.

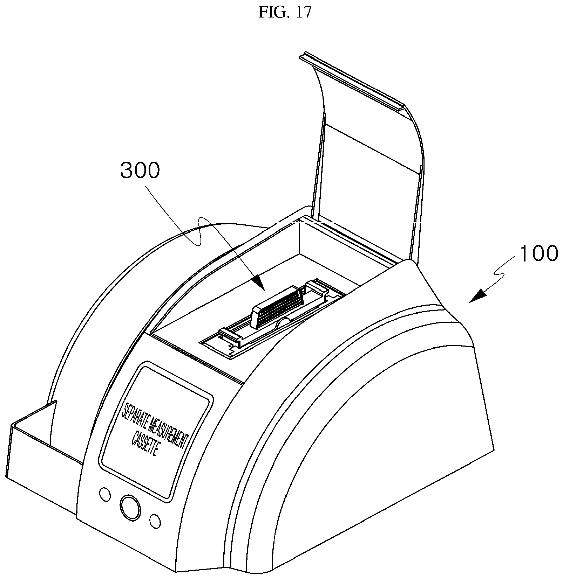

In this case, as illustrated in FIG. 17, the third step (S300) may include displaying a point in time at which the check cassette 300 has to be separated from the inner space S1, but such a step is not necessarily required.

In separating the check cassette 300 from the inner space S1, the fourth step (S400) may be performed only when the check cassette 300 satisfies a predetermined second condition. The predetermined second condition may include various conditions.

For example, the predetermined second condition may be at least one of a condition related to whether the check cassette 300 is completely separated from the inner space S1 and a condition related to time taken for the check cassette 300 to be completely separated from the inner space S1.

First, in a case in which the predetermined second condition is the condition related to whether the check cassette 300 is completely separated from the inner space S1, the check cassette 300 has to reach a state illustrated in FIG. 18 in order to satisfy the predetermined second condition.

Second, in a case in which the predetermined second condition is the condition related to time taken for the check cassette 300 to be completely separated from the inner space S1, time taken for the check cassette 300 to reach the state illustrated in FIG. 18 has to be within a predetermined third time in order to satisfy the predetermined second condition.

For example, in a case in which the predetermined third time is five seconds, when the check cassette 300 is not completely separated from the inner space S1 until five seconds passes from a point in time at which the user is informed of the fact that the current point in time is a point in time at which the check cassette 300 has to be separated from the inner space S1, the predetermined second condition is not satisfied.

In this case, the measurement device 100 may output a signal that allows the non-satisfaction state to be identified visually or aurally by the controller.

To determine the above-described predetermined third condition, the measurement device 100 according to the present disclosure may include a detection means such as a sensor capable of sensing whether the check cassette 300 has been completely separated from the inner space S1, a timer, and the like.

After the predetermined second condition is satisfied and the third step is performed, the fourth step (S400) in which the check cassette 300 is inserted into the inner space S1 in a second state, which differs from the first state, may be performed.

As described above, the second state may be a state in which the check cassette 300 is rotated 180° from the first state, and the fourth step (S400) may include displaying a point in time at which the check cassette 300 is inserted into the inner space S1, but such a step is not necessarily required.

In inserting the check cassette 300 into the inner space S1 in the second state, the fourth step (S400) may be performed only when the check cassette 300 satisfies a predetermined third condition. The predetermined third condition may include various conditions.

For example, the predetermined third condition may be at least one of a condition related to whether the check cassette 300 is completely inserted into the inner space S1 in the second state, a condition related to time taken for the check cassette 300 to be completely inserted into the inner space S1, and a condition related to time during which the check cassette 300 remains completely inserted into the inner space S1.

First, in a case in which the predetermined third condition is the condition related to whether the check cassette 300 is completely inserted into the inner space S1 in the second state, the check cassette 300 has to reach the state illustrated in FIG. 15 (in this case, the check cassette 300 is inserted into the inner space S1 in the second state) in order to satisfy the predetermined third condition.

Second, in a case in which the predetermined third condition is the condition related to time taken for the check cassette 300 to be completely inserted into the inner space S1, time taken for the check cassette 300 to reach the state illustrated in FIG. 15 (in this case, the check cassette 300 is inserted into the inner space S1 in the second state) has to be within a predetermined fourth time in order to satisfy the predetermined third condition.

For example, in a case in which the predetermined fourth time is five seconds, when the check cassette 300 is not completely inserted into the inner space S1 until five seconds passes from a point in time at which a point in time at which the check cassette 300 is inserted into the inner space S1 is displayed, the predetermined third condition is not satisfied.

In this case, the measurement device 100 may output a signal that allows the non-satisfaction state to be identified visually or aurally by the controller.

Third, in a case in which the predetermined third condition is the condition related to time during which the check cassette 300 remains completely inserted into the inner space S1, the check cassette 300 has to remain in the state illustrated in FIG. 15 (in this case, the check cassette 300 is inserted into the inner space S1 in the second state) during a predetermined fifth time in order to satisfy the predetermined third condition.

For example, in a case in which the predetermined fifth time is five seconds, when the check cassette 300 does not remain completely inserted into the inner space S1 for at least five seconds, the predetermined third condition is not satisfied.

In this case, the measurement device 100 may output a signal that allows the non-satisfaction state to be identified visually or aurally by the controller.

To determine the above-described predetermined third condition, the measurement device 100 according to the present disclosure may include a detection means such as a sensor capable of sensing whether the check cassette 300 has been completely inserted into the inner space S1, a timer, and the like.

After the check cassette 300 satisfies the predetermined third condition and is inserted into the inner space S1 in the second state, the fifth step (S500) in which the second reaction property information of light irradiated from the light source due to the second reaction surface 320 is extracted may be performed.

The fifth step (S500) may be a step in which the second surface 320 becomes a surface located closer to the light source than the first surface 310 such that the second reaction property information of light irradiated from the light source due to the second reaction surface 325 is extracted by the measurement device 100.

Specifically, when the check cassette 300 satisfies the predetermined third condition, the controller may allow light to be irradiated from the light-emitting element toward the second reaction surface 325, and extract the second reaction property information through light intensity of light that is reacted due to the second reaction surface 325 and then reaches the light-receiving element.

In this case, as described above, the second reaction surface 325 has a second specific reaction property regarding specific light. In a case in which light irradiated from the light-emitting element has the same frequency and light intensity as those of specific light, the second specific reaction property and the second reaction property have to be the same.

In other words, in a case in which the specific light is the light of the light source, when the second reaction property differs from the second specific reaction property, this signifies that light intensity of the light source has changed.

In the case in which the check cassette 300 is inserted into the inner space S1 in the second state, the first reaction surface 315 of the first surface 310 may not exhibit a reaction property regarding the light irradiated from the light source.

After the fifth step (S500) is performed, the sixth step (S600) in which a change in light intensity of the light source in accordance with use of the measurement device 100 is compensated for on the basis of the extracted first reaction property information and second reaction property information may be performed.

The controller may compensate for the relationship between light intensity of light irradiated from the light-emitting element and light intensity of light that reaches the light-receiving element in accordance with the amount of glycated hemoglobin on the basis of the first reaction property information and the second reaction property information extracted through the second step (S200) and the fifth step (S500).

In this case, the sixth step (S600) may be performed after the third step (S300) is re-performed after the fifth step (S500) is performed.

In this case, the controller may compensate for a change in light intensity of the light source by using an average value of the first reaction property information and the second reaction property information, but embodiments are not necessarily limited thereto. The controller may also compensate for a change in light intensity of the light source by assigning a weighted value to any one of the first reaction property information and the second reaction property information

The sixth step (S600) may include displaying a point in time at which the check cassette 300 has to be separated from the inner space S1, but such a step is not necessarily required.

Comparing the position of the first reaction surface 315 in the case in which the check cassette 300 is inserted into the inner space S1 in the first state in the first step (S100) and the position of the second reaction surface 325 in the case in which the check cassette 300 is inserted into the inner space S1 in the second state in the fourth step (S400), the positions may be the same.

The position of the first reaction surface 315 with respect to the first surface 310 and the position of the second reaction surface 325 with respect to the second surface 320 may be the same.

The position of the first reaction surface 315 with respect to the first surface 310 may be a position leaned toward one side with respect to a direction in which the check cassette 300 is inserted into the inner space S1 in the first state, and the position of the second reaction surface 325 with respect to the second surface 320 may be a position leaned toward one side with respect to a direction in which the check cassette 300 is inserted into the inner space S1 in the second state.

This is because, since the light source in the measurement device 100 is disposed in a state in which it is fixed to a predetermined position, the first reaction surface 315 has to be stably located at an optimal position regarding the relationship with the light source in the case in which the check cassette 300 is inserted into the inner space S1 in the first state, and likewise, the second reaction surface 325 has to be stably located at an optimal position regarding the relationship with the light source in the case in which the check cassette 300 is inserted into the inner space S1 in the second state.

In the case in which the first reaction property information is extracted through the second step (S200), the second reaction surface 325 of the second surface 320 may not exhibit a reaction property regarding light irradiated from the light source due to the positional relationship between the light source and the second reaction surface 325, and likewise, in the case in which the second reaction property information is extracted through the fifth step (S500), the first reaction surface 315 of the first surface 310 may not exhibit a reaction property regarding light irradiated from the light source.

As described above with reference to FIGS. 1 to 9, the measurement device 100 includes the measurement cassette guide portion 130 configured to allow the check cassette 300 to be inserted into the inner space S1 only in a specific state.

However, in the cases in which the check cassette 300 is inserted into the inner space S1 in the first state and the second state through the first step (S100) and the fourth step (S400), the measurement cassette guide portion 130 may not interfere with the check cassette 300.

This is because, unlike the measurement cassette 200, the check cassette 300 has to be inserted into the inner space S1 in both the first state and the second state.

The above-described method for compensating for light intensity of a light source for a measurement device according to the present disclosure may be implemented in the form of a code or program performing the method, and such a code or program may be stored in a computer-readable recording medium.

A means in which the above-described method is stored in the form of a program in a readable recording medium and distributed may include the following examples.

A program for performing the above-described methods may be recorded in a portable recording medium such as a compact disk (CD)-read only memory (ROM), a floppy disk, and a flash memory, and the portable recording medium may be distributed.

Alternatively, a server including a recording medium (that is, a storage unit such as a hard disk) in which a program for performing the above-described methods is recorded may be provided, and in response to requests from users, the program may send a code related to the above-described program through the server to electronic devices such as smartphones and/or desktops of the users so that the program is distributed.

Alternatively, an electronic device which includes a recording medium (that is, a built-in memory) that contains a program for performing the above-described methods may be manufactured by a manufacturer of electronic devices and the like, and such an electronic device may be distributed so that the program is distributed.

### (2) Second example, not falling under the scope of the claims

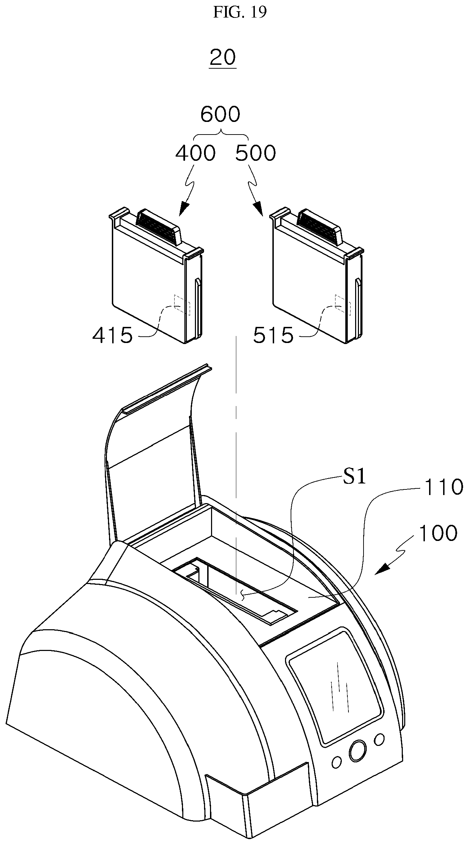

FIG. 19 is a schematic perspective view illustrating a system for compensating for light intensity of a light source for a measurement device according to the present disclosure.

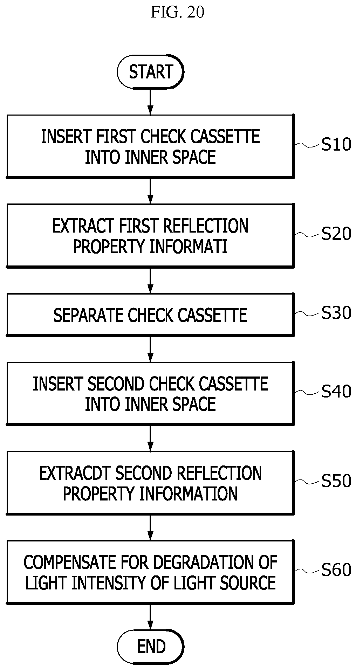

FIG. 20 is a flowchart for describing a method for compensating for light intensity of a light source for a measurement device by using a first check cassette and a second check cassette according to FIG. 19, and FIGS. 21 to 26 are views for describing each step according to FIG. 20 in detail.

First, referring to FIG. 19, a system 20 for compensating for light intensity of a light source for a measurement device according to the present disclosure may include the measurement device 100, which has been described above with reference to FIGS. 1 to 9, and a check cassette 600. Details related to the check cassette 600 which have already been described above will be omitted.

The check cassette 600 may include a first check cassette 400 and a second check cassette 500. Unlike the measurement cassette 200 described with reference to FIGS. 1 to 9, the first check cassette 400 and the second check cassette 500 may be cassettes for compensating for a change in light intensity of the measurement device 100.

The first check cassette 400 may include a first reaction surface 415 having a first specific reaction property regarding specific light, and the second check cassette 500 may include a second reaction surface 515 having a second specific reaction property regarding the specific light.

The measurement device 100 may include a main body portion 110 and a controller.

The controller may extract first reaction property information of light irradiated from the light source due to the first reaction surface 415 when the first check cassette 400 is inserted into the inner space S1 in accordance with a predetermined first condition, extract second reaction property information of light irradiated from the light source due to the second reaction surface 515 when the second check cassette 500 is inserted into the inner space S1 in accordance with a predetermined third condition, and compensate for a change in light intensity of the light source in accordance with use of the measurement device 100 on the basis of the extracted first reaction property information and the second reaction property information.

In this case, when the first check cassette 400 satisfies the predetermined first condition including at least one of a condition related to a state of a direction in which the first check cassette 400 is inserted into the inner space S1, a condition related to whether the first check cassette 400 is completely inserted in to the inner space, a condition related to time taken for the first check cassette 400 to be completely inserted into the inner space, and a condition related to time during which the first check cassette 400 remains completely inserted into the inner space, the controller may extract the first reaction property information of light irradiated from the light source due to the first reaction surface 415.

After extracting the first reaction property information, the controller may extract the second reaction property information when the first check cassette 400 satisfies a predetermined second condition including at least one of a condition related to whether the first check cassette 400 is completely separated from the inner space and a condition related to time taken for the first check cassette 400 to be completely separated from the inner space.

When the second check cassette 500 satisfies the predetermined second condition including at least one of a condition related to a state of a direction in which the second check cassette 500 is inserted into the inner space S1, a condition related to whether the second check cassette 500 is completely inserted in to the inner space, a condition related to time taken for the second check cassette 500 to be completely inserted into the inner space, and a condition related to time during which the second check cassette 500 remains completely inserted into the inner space, the controller may extract the second reaction property information of light irradiated from the light source due to the second reaction surface 515.

Referring to FIG. 20, a method for compensating for light intensity of a light source using a system for compensating for light intensity of a light source for a measurement device according to the present disclosure may include a first step (S10) in which the first check cassette 400 is inserted into the inner space S1 of the measurement device 100, a second step (S20) in which first reaction property information of light irradiated from the light source due to the first reaction surface 415 is extracted, a third step (S30) in which the first check cassette 400 is separated from the inner space S1, a fourth step (S40) in which the second check cassette 500 is inserted into the inner space S1, a fifth step (S50) in which second reaction property information of light irradiated from the light source due to the second reaction surface 515 is extracted, and a sixth step (S60) in which a change in light intensity of the light source in accordance with use of the measurement device 100 is compensated for on the basis of the extracted first reaction property information and second reaction property information.

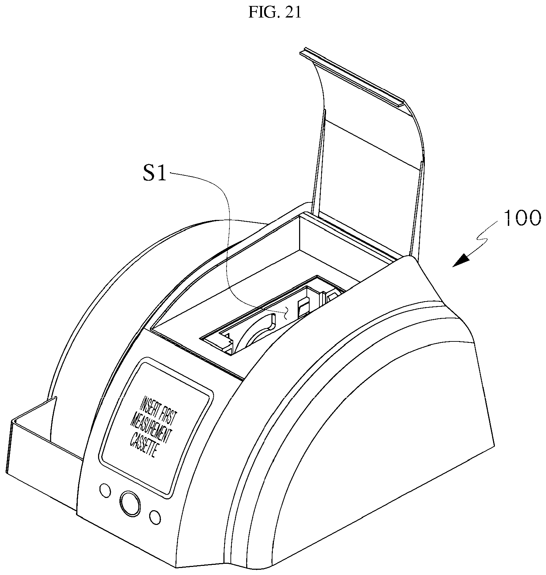

Hereinafter, each step will be described in more detail with reference to FIGS. 21 to 26.

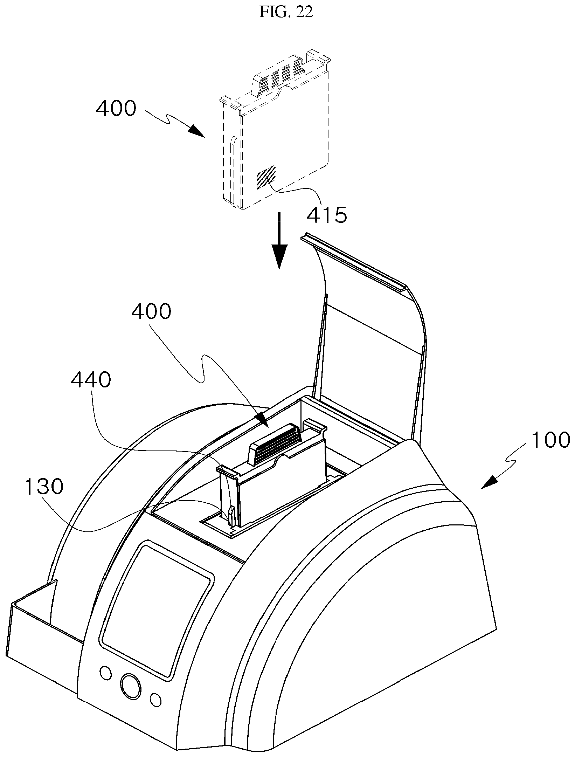

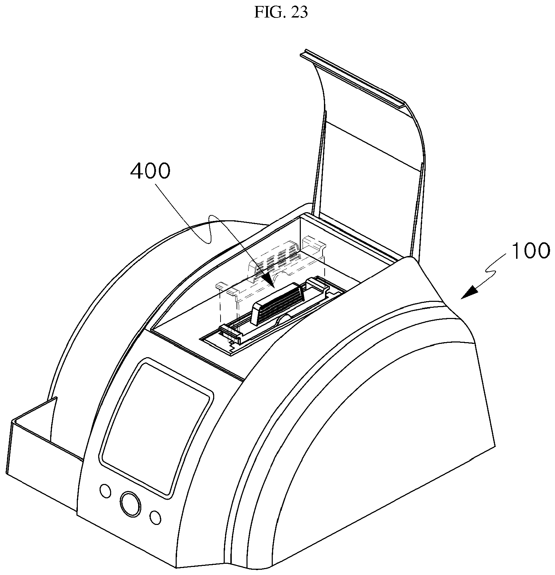

Referring to FIGS. 22 and 23, the first step (S10) in which the first check cassette 400, which includes the first reaction surface 415 having the first specific reaction property regarding specific light, is inserted into the inner space S1 may be performed.

In this case, as illustrated in FIG. 21, as described above with reference to FIG. 13, to insert the first check cassette 400 into the inner space S1, selecting a mode of the measurement device 100 and displaying a point in time at which the first check cassette 400 is inserted may be performed in the first step (S10). Detailed description thereof will be omitted.

In inserting the first check cassette 400 into the inner space S1, the second step (S20) may be performed only when the first check cassette 400 satisfies a predetermined first condition. The predetermined first condition may include various conditions.

For example, the predetermined first condition may be at least one of a condition related to whether the first check cassette 400 is completely inserted into the inner space S1, a condition related to time taken for the first check cassette 400 to be completely inserted into the inner space S1, and a condition related to time during which the first check cassette 400 remains completely inserted into the inner space S1.

First, in a case in which the predetermined first condition is the condition related to whether the first check cassette 400 is completely inserted into the inner space S1, the first check cassette 400 has to reach a state illustrated in FIG. 23 in order to satisfy the predetermined first condition.

In this case, for the first check cassette 400 to reach the state illustrated in FIG. 23, a corresponding portion 440 of the first check cassette 400 has to be inserted into the measurement cassette guide portion 130 of the measurement device 100. Detailed description thereof will be omitted.

Second, in a case in which the predetermined first condition is the condition related to time taken for the first check cassette 400 to be completely inserted into the inner space S1, time taken for the first check cassette 400 to reach the state illustrated in FIG. 23 has to be within a predetermined first time in order to satisfy the predetermined first condition.

For example, in a case in which the predetermined first time is five seconds, when the first check cassette 400 is not completely inserted into the inner space S1 until five seconds passes from a point in time at which the first check cassette 400 has to be inserted into the inner space S1 after the check mode is selected, the predetermined first condition is not satisfied.

In this case, the measurement device 100 may output a signal that allows the non-satisfaction state to be identified visually or aurally by a controller.

Third, in a case in which the predetermined first condition is the condition related to time during which the first check cassette 400 remains completely inserted into the inner space S1, the first check cassette 400 has to remain in the state illustrated in FIG. 23 during a predetermined second time in order to satisfy the predetermined first condition.

For example, in a case in which the predetermined second time is five seconds, when the first check cassette 400 does not remain completely inserted into the inner space S1 for at least five seconds, the predetermined first condition is not satisfied.

In this case, the measurement device 100 may output a signal that allows the non-satisfaction state to be identified visually or aurally by the controller.

To determine the above-described predetermined first condition, the measurement device 100 according to the present disclosure may include a detection means such as a sensor capable of sensing whether the first check cassette 400 has been completely inserted into the inner space S1, a timer, and the like.

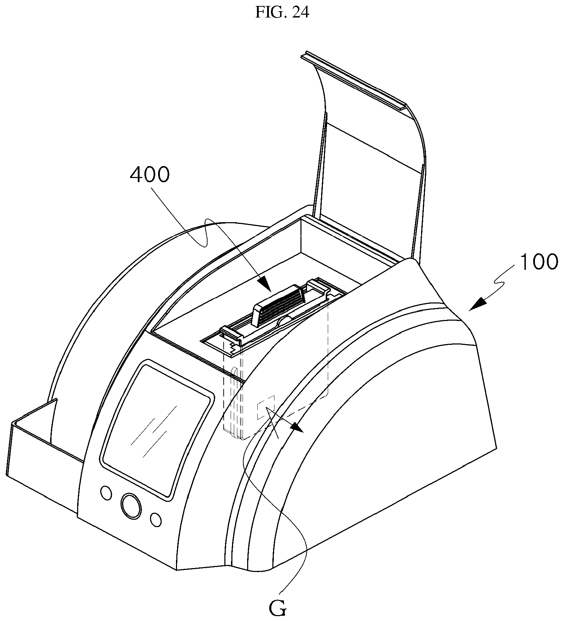

Referring to FIG. 24, after the first check cassette 400 is inserted into the inner space S1, the second step (S20) in which first reaction property information of light G irradiated from the light source due to the first reaction surface 415 is extracted may be performed.

Specifically, when the first check cassette 400 satisfies the predetermined first condition, the controller may allow the light G to be irradiated from a light-emitting element toward the first reaction surface 415, and extract the first reaction property information through light intensity of light that is reacted due to the first reaction surface 415 and then reaches the light-receiving element.

In this case, as described above, the first reaction surface 415 has a first specific reaction property regarding specific light. In a case in which light irradiated from the light-emitting element has the same frequency and light intensity as those of specific light, the first specific reaction property and the first reaction property have to be the same.

In other words, in a case in which the specific light is the light of the light source, when the first reaction property differs from the first specific reaction property, this signifies that light intensity of the light source has changed.

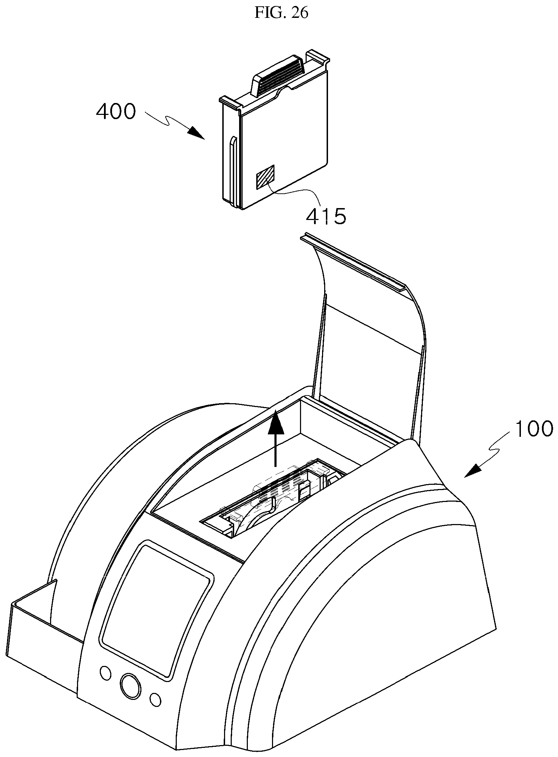

Referring to FIG. 26, after the first reaction property information of light irradiated from the light source due to the first reaction surface 415 is extracted, the third step (S30) in which the first check cassette 400 is separated from the inner space S1 may be performed.

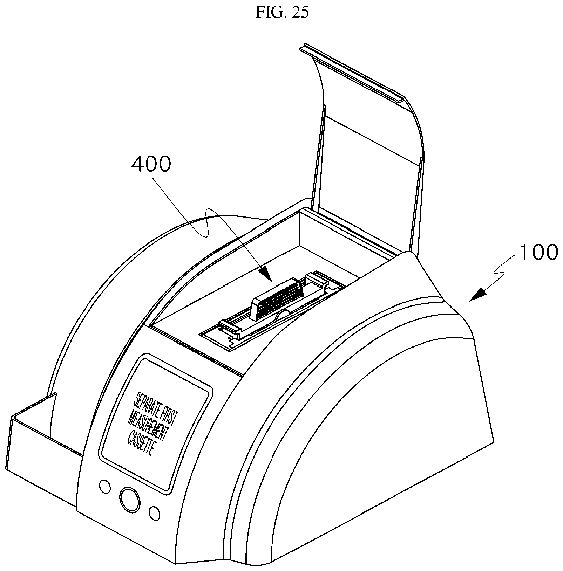

In this case, as illustrated in FIG. 25, the third step (S30) may include displaying a point in time at which the first check cassette 400 has to be separated from the inner space S1, but such a step is not necessarily required.

In separating the first check cassette 400 from the inner space S1, the fourth step (S400) may be performed only when the first check cassette 400 satisfies a predetermined second condition. The predetermined second condition may include various conditions.

For example, the predetermined second condition may be at least one of a condition related to whether the first check cassette 400 is completely separated from the inner space S1 and a condition related to time taken for the first check cassette 400 to be completely separated from the inner space S1.

First, in a case in which the predetermined second condition is the condition related to whether the first check cassette 400 is completely separated from the inner space S1, the first check cassette 400 has to reach a state illustrated in FIG. 26 in order to satisfy the predetermined second condition.

Second, in a case in which the predetermined second condition is the condition related to time taken for the first check cassette 400 to be completely separated from the inner space S1, time taken for the first check cassette 400 to reach the state illustrated in FIG. 26 has to be within a predetermined third time in order to satisfy the predetermined second condition.

For example, in a case in which the predetermined third time is five seconds, when the first check cassette 400 is not completely separated from the inner space S1 until five seconds passes from a point in time at which the user is informed of the fact that the current point in time is a point in time at which the first check cassette 400 has to be separated from the inner space S1, the predetermined third condition is not satisfied.

In this case, the measurement device 100 may output a signal that allows the non-satisfaction state to be identified visually or aurally by the controller.

To determine the above-described predetermined third condition, the measurement device 100 according to the present disclosure may include a detection means such as a sensor capable of sensing whether the first check cassette 400 has been completely separated from the inner space S1, a timer, and the like.

After the predetermined second condition is satisfied and the third step is performed, the fourth step (S40) in which the second check cassette 500 is inserted into the inner space S1 may be performed.

The fourth step (S40) may include displaying a point in time at which the second check cassette 500 is inserted into the inner space S1, but such a step is not necessarily required.

In inserting the second check cassette 500 into the inner space S1, the fourth step (S40) may be performed only when the second check cassette 500 satisfies a predetermined third condition. The predetermined third condition may include various conditions.

For example, the predetermined third condition may be at least one of a condition related to whether the second check cassette 500 is completely inserted into the inner space S1, a condition related to time taken for the second check cassette 500 to be completely inserted into the inner space S1, and a condition related to time during which the second check cassette 500 remains completely inserted into the inner space S1.

First, in a case in which the predetermined third condition is the condition related to whether the second check cassette 500 is completely inserted into the inner space S1, the second check cassette 500 has to reach the state illustrated in FIG. 26 (in this case, the check cassette 600 is the second check cassette 500) in order to satisfy the predetermined third condition.

Second, in a case in which the predetermined third condition is the condition related to time taken for the second check cassette 500 to be completely inserted into the inner space S1, time taken to reach the state illustrated in FIG. 26 (in this case, the check cassette 600 is the second check cassette 500) has to be within a predetermined fourth time in order to satisfy the predetermined third condition.

For example, in a case in which the predetermined fourth time is five seconds, when the second check cassette 500 is not completely inserted into the inner space S1 until five seconds passes from a point in time at which a point in time at which the second check cassette 500 is inserted into the inner space S1 is displayed, the predetermined third condition is not satisfied.

In this case, the measurement device 100 may output a signal that allows the non-satisfaction state to be identified visually or aurally by the controller.

Third, in a case in which the predetermined third condition is the condition related to time during which the second check cassette 500 remains completely inserted into the inner space S1, the state illustrated in FIG. 26 (in this case, the check cassette 600 is the second check cassette 500) has to be maintained during a predetermined fifth time in order to satisfy the predetermined third condition.

For example, in a case in which the predetermined fifth time is five seconds, when the second check cassette 500 does not remain completely inserted into the inner space S1 for at least five seconds, the predetermined third condition is not satisfied.

In this case, the measurement device 100 may output a signal that allows the non-satisfaction state to be identified visually or aurally by the controller.

To determine the above-described predetermined third condition, the measurement device 100 according to the present disclosure may include a detection means such as a sensor capable of sensing whether the second check cassette 500 has been completely inserted into the inner space S1, a timer, and the like.

After the second check cassette 500 satisfies the predetermined third condition and is inserted into the inner space S1, the fifth step (S500) in which the second reaction property information of light irradiated from the light source due to the second reaction surface 515 is extracted may be performed.

Specifically, when the second check cassette 500 satisfies the predetermined third condition, the controller may allow light to be irradiated from the light-emitting element toward the second reaction surface 515, and extract the second reaction property information through light intensity of light that is reacted due to the second reaction surface 515 and then reaches the light-receiving element.

In this case, as described above, the second reaction surface 515 has a second specific reaction property regarding specific light. In a case in which light irradiated from the light-emitting element has the same frequency and light intensity as those of specific light, the second specific reaction property and the second reaction property have to be the same.

In other words, in a case in which the specific light is the light of the light source, when the second reaction property differs from the second specific reaction property, this signifies that light intensity of the light source has changed.

After the fifth step (S50) is performed, the sixth step (S60) in which a change in light intensity of the light source in accordance with use of the measurement device 100 is compensated for on the basis of the extracted first reaction property information and second reaction property information may be performed.

In this case, the sixth step (S60) may be performed after separating of the second check cassette 500, which is the same as the third step (S30), is performed after the fifth step (S50) is performed.

The controller may compensate for the relationship between light intensity of light irradiated from the light-emitting element and light intensity of light that reaches the light-receiving element in accordance with the amount of glycated hemoglobin on the basis of the first reaction property information and the second reaction property information extracted through the second step (S20) and the fifth step (S50).

In this case, the controller may compensate for a change in light intensity of the light source by using an average value of the first reaction property information and the second reaction property information, but embodiments are not necessarily limited thereto. The controller may also compensate for a change in light intensity of the light source by assigning a weighted value to any one of the first reaction property information and the second reaction property information

The sixth step (S60) may include displaying a point in time at which the second check cassette 500 has to be separated from the inner space S1, but such a step is not necessarily required.

Comparing the position of the first reaction surface 415 in the case in which the first check cassette 400 is inserted into the inner space S1 in the first step (S10) and the position of the second reaction surface 515 in the case in which the second check cassette 500 is inserted into the inner space S1 in the fourth step (S40), the positions may be the same.

The position of the first reaction surface 415 may be a position leaned toward one side with respect to a direction in which the first check cassette 400 is inserted into the inner space S1, and the position of the second reaction surface 515 may be a position leaned toward one side with respect to a direction in which the second check cassette 500 is inserted into the inner space S1.

This is because, since the light source in the measurement device 100 is disposed in a state in which it is fixed to a predetermined position, the first reaction surface 415 has to be stably located at an optimal position regarding the relationship with the light source in the case in which the first check cassette 400 is inserted into the inner space S1, and likewise, the second reaction surface 515 has to be stably located at an optimal position regarding the relationship with the light source in the case in which the second check cassette 500 is inserted into the inner space S1.

The above-described method for compensating for light intensity of a light source for a measurement device according to the present disclosure may be implemented in the form of a code or program performing the method, and such a code or program may be stored in a computer-readable recording medium.

A means in which the above-described method is stored in the form of a program in a readable recording medium and distributed may include the following examples.

A program for performing the above-described methods may be recorded in a portable recording medium such as a CD-ROM, a floppy disk, and a flash memory, and the portable recording medium may be distributed.

Alternatively, a server including a recording medium (that is, a storage unit such as a hard disk) in which a program for performing the above-described methods is recorded may be provided, and in response to requests from users, the program may send a code related to the above-described program through the server to electronic devices such as smartphones and/or desktops of the users so that the program is distributed.

Alternatively, an electronic device which includes a recording medium (that is, a built-in memory) that contains a program for performing the above-described methods may be manufactured by a manufacturer of electronic devices and the like, and such an electronic device may be distributed so that the program is distributed.

Configurations and features of the present disclosure have been described above on the basis of embodiments according to the present disclosure. The invention is only limited by the appended claims.

For example, the configuration related to the pressing force applied to the measurement cassette 200, which has been described above with reference to FIGS. 1 to 8, may be identically applied to the check cassettes 300 and 600.

## Claims

1. A system for compensating light intensity of a light source for measuring an analyte of a biological sample on the basis of reaction properties of light irradiated from the light source, the system comprising:
a measurement device (100) having a light source, a light receiving element, a controller and an inner space for receiving a cassette; and
a check cassette (300) having at least two reaction surfaces (315, 325) on the opposite surfaces that is used in the measurement device so as to compensate the light intensity of the light source,
**characterized in that** the check cassette comprises:
a first surface (315) which has a first reaction surface having a first specific reaction property to specific light wherein the first specific reaction property of the first reaction surface to the specific light is measured on the basis of reaction properties of light irradiated from the light source of the measurement device including at least one of reflection, absorption, and transmission of the light; and
a second surface (325) which is disposed opposite the first surface and has a second reaction surface having a second specific reaction property to the specific light wherein the second specific reaction property of the second reaction surface to the specific light is measured on the basis of reaction properties of light irradiated from a light source of the measurement device including at least one of reflection, absorption, and transmission of the light,
wherein information of the first reaction property of the first reaction surface is measured only when the check cassette is inserted into the inner space of the measurement device in a first state of one direction, and information of the second reaction property of the second reaction surface is measured only when the check cassette is inserted into the inner space of the measurement device in a second state of the other direction, and
wherein the controller is configured to compensate the light intensity of the light source of the measurement device on the basis of the information of the first specific reaction property and the information of the second specific reaction property.

2. The system of claim 1, wherein a position of the first reaction surface in the case in which the check cassette is inserted into the inner space in the first state is the same as a position of the second reaction surface in the case in which the check cassette is inserted into the inner space in the second state.

3. The system of claim 1, wherein a position of the first reaction surface with respect to the first surface is the same as a position of the second reaction surface with respect to the second surface.

4. The system of claim 1, wherein a position of the first reaction surface with respect to the first surface is leaned toward one side with respect to a direction in which the check cassette is inserted into the inner space in the first state.

5. The system of claim 1, wherein a position of the second reaction surface with respect to the second surface is leaned toward one side with respect to a direction in which the check cassette is inserted into the inner space in the second state.

6. The system of claim 1, wherein the second state is a state in which the check cassette is rotated 180° from the first state with respect to a direction in which the check cassette is inserted into the inner space in the first state.

7. The system of claim 1, wherein the measurement device includes a measurement cassette guide portion configured to allow a measurement cassette to be inserted into the inner space only in a specific state,
in the cases in which the check cassette is inserted into the inner space in the first state and the second state, the measurement cassette guide portion does not interfere with the check cassette.

8. A method for compensating light intensity of a light source for a measurement device for measurement of an analyte of a biological sample, the method comprising:
a first step (S100) in which a check cassette, which includes a first surface which has a first reaction surface having a first specific reaction property to specific light and a second surface which is disposed opposite the first surface and has a second reaction surface having a second specific reaction property to the specific light, is inserted into the inner space in a first state of a first direction;
a second step (S200) in which, a first reaction property information of the first reaction surface to a specific light of the light source is measured on the basis of reaction properties of light irradiated from the light source of the measurement device including at least one of reflection, absorption, and transmission of the light;
a third step (S300) in which the check cassette is separated from the inner space;
a fourth step (S400) in which the check cassette is inserted into the inner space in a second state of a second direction, which differs from the first direction;
a fifth step (S500) in which, when the check cassette is inserted into the inner space in the second state, second reaction property information of the second reaction surface to the specific light of the light source is measured on the basis of reaction properties of light irradiated from the light source of the measurement device including at least one of reflection, absorption, and transmission of the light; and
a sixth step (S600) in which light intensity of the light source in accordance with use of the measurement device is compensated on the basis of the measured first reaction property information and the measured second reaction property information.

9. The method of claim 8, wherein the second state is a state in which the check cassette is rotated 180° from the first state with respect to a direction in which the check cassette is inserted into the inner space in the first state.

10. The method of claim 8, wherein the first step includes, when the check cassette does not satisfy a predetermined first condition including at least one of a condition related to whether the check cassette is completely inserted into the inner space in the first state, a condition related to time taken for the check cassette to be completely inserted into the inner space, and a condition related to time during which the check cassette remains completely inserted into the inner space, outputting a signal that allows the non-satisfaction state to be identified.

11. The method of claim 8, wherein the third step includes, when the check cassette does not satisfy a predetermined second condition including at least one of a condition related to whether the check cassette is completely separated from the inner space and a condition related to time taken for the check cassette to be completely separated, outputting a signal that allows the non-satisfaction state to be identified.

12. The method of claim 8, wherein the fourth step includes, when the check cassette does not satisfy a predetermined third condition including at least one of a condition related to whether the check cassette is completely inserted into the inner space in the second direction, a condition related to time taken for the check cassette to be completely inserted into the inner space, and a condition related to time during which the check cassette remains completely inserted into the inner space, outputting a signal that allows the non-satisfaction state to be identified.

13. The method of claim 8, wherein the first step includes selecting a mode of the measurement device in order to insert the check cassette into the inner space in the first direction.

## Patentansprüche

1. System zum Kompensieren der Lichtintensität einer Lichtquelle zum Messen eines Analyten einer biologischen Probe auf der Grundlage von Reaktionseigenschaften von Licht, das von der Lichtquelle ausgestrahlt wird, wobei das System umfasst:
eine Messvorrichtung (100) mit einer Lichtquelle, einem Lichtempfangselement, einem Steuergerät und einem Innenraum zum Aufnehmen einer Kassette; und
eine Prüfkassette (300) mit mindestens zwei Reaktionsflächen (315, 325) auf den gegenüberliegenden Flächen, die in der Messvorrichtung verwendet wird, um die Lichtintensität der Lichtquelle zu kompensieren,
**dadurch gekennzeichnet, dass** die Prüfkassette umfasst:
eine erste Fläche (315), die eine erste Reaktionsfläche mit einer ersten spezifischen Reaktionseigenschaft gegenüber spezifischem Licht aufweist, wobei die erste spezifische Reaktionseigenschaft der ersten Reaktionsfläche gegenüber dem spezifischem Licht auf der Grundlage von Reaktionseigenschaften von Licht gemessen wird, das von der
Lichtquelle der Messvorrichtung ausgestrahlt wird, einschließlich mindestens eine von Reflexion, Absorption und Übertragung des Lichts; und
eine zweite Fläche (325), die gegenüber der ersten Fläche angeordnet ist und eine zweite Reaktionsfläche mit einer zweiten spezifischen Reaktionseigenschaft gegenüber dem spezifischem Licht aufweist, wobei die zweite spezifische Reaktionseigenschaft der zweiten Reaktionsfläche gegenüber dem spezifischem Licht auf der Grundlage von Reaktionseigenschaften von Licht gemessen wird, das von einer Lichtquelle der Messvorrichtung ausgestrahlt wird, einschließlich mindestens einer von Reflexion, Absorption und Übertragung des Lichts,
wobei Informationen der ersten Reaktionseigenschaft der ersten Reaktionsfläche nur gemessen werden, wenn die Prüfkassette in einem ersten Zustand einer Richtung in den Innenraum der Messvorrichtung eingeführt wird, und Informationen der zweiten Reaktionseigenschaft der zweiten Reaktionsfläche nur gemessen werden, wenn die Prüfkassette in einem zweiten Zustand der anderen Richtung in den Innenraum der Messvorrichtung eingeführt wird, und
wobei das Steuergerät konfiguriert ist, um die Lichtintensität der Lichtquelle der Messvorrichtung auf der Grundlage der Informationen der ersten spezifischen Reaktionseigenschaft und der Informationen der zweiten spezifischen Reaktionseigenschaft zu kompensieren.

2. System nach Anspruch 1, wobei eine Position der ersten Reaktionsfläche in dem Fall, in dem die Prüfkassette in dem ersten Zustand in den Innenraum eingeführt wird, dieselbe ist wie eine Position der zweiten Reaktionsfläche in dem Fall, in dem der Prüfkassette in dem zweiten Zustand in den Innenraum eingeführt wird.

3. System nach Anspruch 1, wobei eine Position der ersten Reaktionsfläche in Bezug auf die erste Fläche dieselbe ist wie eine Position der zweiten Reaktionsfläche in Bezug auf die zweite Fläche.

4. System nach Anspruch 1, wobei eine Position der ersten Reaktionsfläche in Bezug auf die erste Fläche zu einer Seite in Bezug auf eine Richtung geneigt ist, in der die Prüfkassette in dem ersten Zustand in den Innenraum eingeführt wird.

5. System nach Anspruch 1, wobei eine Position der zweiten Reaktionsfläche in Bezug auf die zweite Fläche zu einer Seite in Bezug auf eine Richtung geneigt ist, in der die Prüfkassette in dem zweiten Zustand in den Innenraum eingeführt wird.

6. System nach Anspruch 1, wobei der zweite Zustand ein Zustand ist, in dem die Prüfkassette um 180° von dem ersten Zustand in Bezug auf eine Richtung gedreht ist, in der die Prüfkassette in dem ersten Zustand in den Innenraum eingeführt wird.

7. System nach Anspruch 1, wobei die Messvorrichtung einen Messkassetten-Führungsabschnitt einschließt, der konfiguriert ist, um zu ermöglichen, dass eine Messkassette nur in einem bestimmten Zustand in den Innenraum eingeführt wird, in den Fällen, in denen die Prüfkassette in dem ersten Zustand und dem zweiten Zustand in den Innenraum eingeführt wird, der Messkassetten-Führungsabschnitt nicht mit der Prüfkassette störend eingreift.

8. Verfahren zum Kompensieren der Lichtintensität einer Lichtquelle für eine Messvorrichtung zur Messung eines Analyten einer biologischen Probe, wobei das Verfahren umfasst: einen ersten Schritt (S 100), in dem eine Prüfkassette, die eine erste Fläche, die eine erste Reaktionsfläche mit einer ersten spezifischen Reaktionseigenschaft gegenüber spezifischem Licht aufweist, und eine zweite Fläche einschließt, die gegenüber der ersten Fläche angeordnet ist und eine zweite Reaktionsfläche mit einer zweiten spezifischen Reaktionseigenschaft gegenüber spezifischem Licht aufweist, in einem ersten Zustand einer ersten Richtung in den Innenraum eingeführt wird;
einen zweiten Schritt (S200), in dem eine erste Reaktionseigenschaftsinformation der ersten Reaktionsfläche gegenüber spezifischem Licht der Lichtquelle auf der Grundlage von Reaktionseigenschaften von Licht gemessen wird, das von der Lichtquelle der Messvorrichtung ausgestrahlt wird, einschließlich mindestens einer von Reflexion, Absorption und Übertragung des Lichts;
einen dritten Schritt (S300), in dem die Prüfkassette von dem Innenraum getrennt wird;
einen vierten Schritt (S400), in dem die Prüfkassette in einem zweiten Zustand einer zweiten Richtung, die sich von der ersten Richtung unterscheidet, in den Innenraum eingeführt wird;
einen fünften Schritt (S500), in dem, wenn die Prüfkassette in dem zweiten Zustand in den Innenraum eingeführt wird, zweite Reaktionseigenschaftsinformationen der zweiten Reaktionsfläche gegenüber spezifischem Licht der Lichtquelle auf der Grundlage von Reaktionseigenschaften von Licht gemessen werden, das von der Lichtquelle der Messvorrichtung ausgestrahlt wird, einschließlich mindestens einer von Reflexion, Absorption und Übertragung des Lichts; und
einen sechsten Schritt (S600), in dem die Lichtintensität der Lichtquelle gemäß der Verwendung der Messvorrichtung auf der Grundlage der gemessenen ersten Reaktionseigenschaftsinformationen und der gemessenen zweiten Reaktionseigenschaftsinformationen kompensiert wird.

9. Verfahren nach Anspruch 8, wobei der zweite Zustand ein Zustand ist, in dem die Prüfkassette um 180° von dem ersten Zustand in Bezug auf eine Richtung gedreht ist, in der die Prüfkassette in dem ersten Zustand in den Innenraum eingeführt wird.

10. Verfahren nach Anspruch 8, wobei der erste Schritt, wenn die Prüfkassette eine vorbestimmte erste Bedingung nicht erfüllt, einschließlich mindestens einer von einer Bedingung, die sich darauf bezieht, ob die Prüfkassette in dem ersten Zustand vollständig in den Innenraum eingeführt wird, einer Bedingung, die sich auf den Zeitaufwand bezieht, damit die Prüfkassette vollständig in den Innenraum eingeführt wird, und einer Bedingung, die sich auf die Zeit bezieht, während der die Prüfkassette vollständig in den Innenraum eingeführt bleibt, das Ausgeben eines Signals einschließt, das ermöglicht, dass der Nicht-Erfüllungszustand identifiziert wird.

11. Verfahren nach Anspruch 8, wobei der dritte Schritt, wenn die Prüfkassette eine vorbestimmte zweite Bedingung nicht erfüllt, einschließlich mindestens einer von einer Bedingung, die sich darauf bezieht, ob die Prüfkassette vollständig von dem Innenraum getrennt ist, und einer Bedingung, die sich auf den Zeitaufwand bezieht, damit die Prüfkassette vollständig getrennt ist, das Ausgeben eines Signals einschließt, das ermöglicht, dass der Nicht-Erfüllungszustand identifiziert wird.

12. Verfahren nach Anspruch 8, wobei der vierte Schritt, wenn die Prüfkassette eine vorbestimmte dritte Bedingung nicht erfüllt, einschließlich mindestens einer von einer Bedingung, die sich darauf bezieht, ob die Prüfkassette in der zweiten Richtung vollständig in den Innenraum eingeführt wird, einer Bedingung, die sich auf den Zeitaufwand bezieht, damit die Prüfkassette vollständig in den Innenraum eingeführt wird, und einer Bedingung, die sich auf die Zeit bezieht, während der die Prüfkassette vollständig in den Innenraum eingeführt bleibt, das Ausgeben eines Signals einschließt, das ermöglicht, dass der Nicht-Erfüllungszustand identifiziert wird.

13. Verfahren nach Anspruch 8, wobei der erste Schritt das Auswählen eines Modus der Messvorrichtung einschließt, um die Prüfkassette in der ersten Richtung in den Innenraum einzuführen.

## Revendications

1. Système pour compenser l'intensité lumineuse d'une source lumineuse pour mesurer un analyte d'un échantillon biologique sur la base des propriétés de réaction d'une lumière irradiée à partir de la source lumineuse, le système comprenant :
un dispositif de mesure (100) ayant une source lumineuse, un élément récepteur de lumière, un contrôleur et un espace interne pour recevoir une cassette ; et
une cassette de contrôle (300) ayant au moins deux surfaces de réaction (315, 325) sur les surfaces opposées qui est utilisée dans le dispositif de mesure afin de compenser l'intensité lumineuse de la source lumineuse,
**caractérisé en ce que** la cassette de contrôle comprend :
une première surface (315) qui a une première surface de réaction ayant une première propriété de réaction spécifique à une lumière spécifique dans laquelle la première propriété de réaction spécifique de la première surface de réaction à la lumière spécifique est mesurée sur la base des propriétés de réaction d'une lumière irradiée à partir de la source lumineuse du dispositif de mesure comprenant au moins l'une parmi la réflexion, l'absorption et la transmission de la lumière ; et
une seconde surface (325) qui est disposée à l'opposé de la première surface et a une seconde surface de réaction ayant une seconde propriété de réaction spécifique à la lumière spécifique dans laquelle la seconde propriété de réaction spécifique de la seconde surface de réaction à la lumière spécifique est mesurée sur la base des propriétés de réaction de la lumière irradiée à partir d'une source lumineuse du dispositif de mesure comprenant au moins l'une parmi la réflexion, l'absorption et la transmission de la lumière,
dans lequel les informations de la première propriété de réaction de la première surface de réaction sont mesurées uniquement lorsque la cassette de contrôle est insérée dans l'espace interne du dispositif de mesure dans un premier état d'une direction, et les informations de la seconde propriété de réaction de la seconde surface de réaction sont mesurées uniquement lorsque la cassette de contrôle est insérée dans l'espace interne du dispositif de mesure dans un second état de l'autre direction, et
dans lequel le contrôleur est configuré pour compenser l'intensité lumineuse de la source lumineuse du dispositif de mesure sur la base des informations de la première propriété de réaction spécifique et des informations de la seconde propriété de réaction spécifique.

2. Système selon la revendication 1, dans lequel une position de la première surface de réaction dans le cas où la cassette de contrôle est insérée dans l'espace interne dans le premier état est la même qu'une position de la seconde surface de réaction dans le cas où la cassette de contrôle est insérée dans l'espace interne dans le second état.

3. Système selon la revendication 1, dans lequel une position de la première surface de réaction par rapport à la première surface est la même qu'une position de la seconde surface de réaction par rapport à la seconde surface.

4. Système selon la revendication 1, dans lequel une position de la première surface de réaction par rapport à la première surface est inclinée vers un côté par rapport à une direction dans laquelle la cassette de contrôle est insérée dans l'espace interne dans le premier état.

5. Système selon la revendication 1, dans lequel une position de la seconde surface de réaction par rapport à la seconde surface est inclinée vers un côté par rapport à une direction dans laquelle la cassette de contrôle est insérée dans l'espace interne dans le second état.

6. Système selon la revendication 1, dans lequel le second état est un état dans lequel la cassette de contrôle est pivotée à 180° depuis le premier état par rapport à une direction dans laquelle la cassette de contrôle est insérée dans l'espace interne dans le premier état.

7. Système selon la revendication 1, dans lequel le dispositif de mesure comprend une partie de guidage de cassette de mesure configurée pour permettre à une cassette de mesure d'être insérée dans l'espace interne uniquement dans un état spécifique,
dans les cas où la cassette de contrôle est insérée dans l'espace interne dans le premier état et le second état, la partie de guidage de la cassette de mesure n'interfère pas avec la cassette de contrôle.

8. Procédé pour compenser l'intensité lumineuse d'une source lumineuse pour un dispositif de mesure pour mesurer un analyte d'un échantillon biologique, le procédé comprenant :
une première étape (S100) dans laquelle une cassette de contrôle, qui comprend une première surface qui a une première surface de réaction ayant une première propriété de réaction spécifique à une lumière spécifique et une seconde surface qui est disposée à l'opposé de la première surface et a une seconde surface de réaction ayant une seconde propriété de réaction spécifique à une lumière spécifique, est insérée dans l'espace interne dans un premier état d'une première direction ;
une deuxième étape (S200) dans laquelle, des premières informations de propriété de réaction de la première surface de réaction à une lumière spécifique de la source lumineuse sont mesurées sur la base des propriétés de réaction de la lumière irradiée depuis la source lumineuse du dispositif de mesure comprenant au moins l'une parmi la réflexion, l'absorption et la transmission de la lumière ;
une troisième étape (S300) dans laquelle la cassette de contrôle est séparée de l'espace interne ;
une quatrième étape (S400) dans laquelle la cassette de contrôle est insérée dans l'espace interne dans un second état d'une seconde direction, qui diffère de la première direction ;
une cinquième étape (S500) dans laquelle, lorsque la cassette de contrôle est insérée dans l'espace interne dans le second état, les secondes informations de propriété de réaction de la seconde surface de réaction à la lumière spécifique de la source lumineuse sont mesurées sur la base des propriétés de réaction de la lumière irradiée à partir de la source lumineuse du dispositif de mesure comprenant au moins l'une parmi la réflexion, l'absorption et la transmission de la lumière ; et
une sixième étape (S600) dans laquelle l'intensité lumineuse de la source lumineuse conformément à l'utilisation du dispositif de mesure est compensée sur la base des premières informations de propriété de réaction mesurées et des secondes informations de propriété de réaction mesurées.

9. Procédé selon la revendication 8, dans lequel le second état est un état dans lequel la cassette de contrôle est pivotée à 180° depuis le premier état par rapport à une direction dans laquelle la cassette de contrôle est insérée dans l'espace interne dans le premier état.

10. Procédé selon la revendication 8, dans lequel la première étape comprend, lorsque la cassette de contrôle ne satisfait pas une première condition prédéterminée comprenant au moins l'une d'une condition relative au fait que la cassette de contrôle est complètement insérée dans l'espace interne dans le premier état, une condition relative au temps mis pour que la cassette de contrôle soit complètement insérée dans l'espace interne, et une condition relative au temps pendant lequel la cassette de contrôle reste complètement insérée dans l'espace interne, la délivrance d'un signal qui permet d'identifier l'état de non-satisfaction.

11. Procédé selon la revendication 8, dans lequel la troisième étape comprend, lorsque la cassette de contrôle ne satisfait pas une deuxième condition prédéterminée comprenant au moins l'une d'une condition relative au fait que la cassette de contrôle est complètement séparée de l'espace interne et une condition relative au temps mis pour que la cassette de contrôle soit complètement séparée, la délivrance d'un signal qui permet d'identifier l'état de non-satisfaction.

12. Procédé selon la revendication 8, dans lequel la quatrième étape comprend, lorsque la cassette de contrôle ne satisfait pas une troisième condition prédéterminée comprenant au moins l'une d'une condition relative au fait que la cassette de contrôle est complètement insérée dans l'espace interne dans la seconde direction, une condition relative au temps mis pour que la cassette de contrôle soit complètement insérée dans l'espace interne, et une condition relative au temps pendant lequel la cassette de contrôle reste complètement insérée dans l'espace interne, la délivrance d'un signal qui permet d'identifier l'état de non-satisfaction.

13. Procédé selon la revendication 8, dans lequel la première étape comprend la sélection d'un mode du dispositif de mesure afin d'insérer la cassette de contrôle dans l'espace interne dans la première direction.
